(19) **Europäisches Patentamt / European Patent Office / Office européen des brevets**

(11) **EP 1 453 966 B1**

(12) **EUROPEAN PATENT SPECIFICATION**

(45) Date of publication and mention of the grant of the patent:
**11.07.2012 Bulletin 2012/28**

(21) Application number: **02791720.2**

(22) Date of filing: **26.11.2002**

(51) Int Cl.:
*C12P 21/02* (2006.01)    *C12N 15/85* (2006.01)
*C07K 14/505* (2006.01)    *C12N 15/90* (2006.01)

(86) International application number:
**PCT/EP2002/013297**

(87) International publication number:
**WO 2003/046187 (05.06.2003 Gazette 2003/23)**

(54) **METHOD FOR PRODUCING A RECOMBINANT POLYPEPTIDE**

METHODE ZUR HERSTELLUNG VON REKOMBINANTEN POLYPEPTIDEN

PROCEDE DE PRODUCTION D'UN POLYPEPTIDE RECOMBINANT

(84) Designated Contracting States:
**AT BE BG CH CY CZ DE DK EE ES FI FR GB GR IE IT LI LU MC NL PT SE SK TR**
Designated Extension States:
**LT LV RO SI**

(30) Priority: **28.11.2001 US 333868 P**

(43) Date of publication of application:
**08.09.2004 Bulletin 2004/37**

(73) Proprietor: **Sandoz AG**
**4056 Basel (CH)**

(72) Inventors:
• **SCHÖRGENDORFER, Kurt**
  **A-6322 Langkampfen (AT)**
• **WINDISCH, Jörg**
  **A-6233 Kramsach (AT)**
• **KUNERT, Renate**
  **A-2232 Deutsch-Wagram (AT)**
• **UNTERLUGGAUER, Florian**
  **A-6330 Kufstein (AT)**

(56) References cited:
**EP-A- 0 319 206    WO-A-96/35718**
**US-A- 4 965 199**

• **YOON SUNG KWAN ET AL: "Influence of reducing agents on the secretion rate of recombinant erythropoietin from CHO cells." BIOTECHNOLOGY LETTERS, vol. 20, no. 2, February 1998 (1998-02), pages 101-104, XP009007462 ISSN: 0141-5492**

**Description**

Field of the Invention

The present invention relates to methods for producing host cells that express recombinant polypeptides, such as human erythropoietin, and methods for the production of such polypeptides. In particular, the invention relates to a two-vector expression system comprising diverse selection markers and methods for using such a system.

Background to the invention

The regulation of erythropoiesis is accomplished mainly by one glycoprotein hormone, erythropoietin (Epo), which was discovered in 1977. At that time, isolation and purification of Epo was hampered by the relative paucity of starting material that is the urine of aplastic anemia patients. Only submicrogram amounts of the purified hormone were available for study.

The first purification approaches were undertaken in the late 1970s using lectins immobilized with agarose in affinity columns. Wheat germ and phytohemagglutinin bound erythropoietin and a crude urinary preparation could be enriched by up to 100 times for Epo.

By the early 1980s the DNA-sequence of human erythropoietin had been sequenced and cloned. Erythropoietin is a single chain glycoprotein with a molecular weight of approximately 30 to 34 kDa. It contains four cysteinyl residues that form two intrachain disulfide bonds between Cys 7 and Cys 161 and between Cys 29 and Cys 33, respectively. The disulfide bridge between Cys 7 and Cys 161 has been shown to be critical for biological activity. Erythropoietin is heavily glycosylated; approximately 35 to 40% of its molecular mass consists of carbohydrate. The amount of carbohydrate can vary between preparations of urine, so this heterogeneity reflects not the state immediately after secretion by the kidney, but the Epo molecule after preparation from urine; it is affected by the method of preparation used. The oligosaccharide structure of the circulating plasma form or forms of erythropoietin has not been determined since these forms have not been isolated. Erythropoietin has three asparagines (Asn 38, Asn 24 and Asn 83) providing N-glycosylation sites, and one serine (Ser 129) for O-linked glycosylation.

Recombinant human erythropoietin (rhEpo) has been expressed in a number of mammalian systems including Chinese hamster ovary (CHO) cells, baby hamster kidney (BHK) cells and HeLa cells. The glycosylation pattern of rhEpo expressed in these different cell types varies. (reviewed in Takeuchi and Kobata, 1991, Glycobiology 1 (4): 337-346). Commercial production of rhEpo requires high level expression of protein that has high *in vivo* activity. One technique for increasing the levels of expression is to transfect a vector containing the dihydrofolate reductase (dhfr) gene in addition to the human Epo gene into dhfr deficient CHO cells (Lee et al., 1999 (J. Biotech 69: 85-93). Addition of methotrexate (MTX) to the culture medium results in amplification of the dhfr gene and also the linked Epo gene.

Summary of the invention

The present invention provides an improved method of producing host cell lines that express recombinant polypeptides such as human erythropoietin. It has been found that introduction into a host cell of two vectors that both contain an expression cassette that directs expression of a recombinant polypeptide, such as rhEpo, in the host cell, one vector containing a gene that is amplified in response to a selection agent (such as dhfr) and the other vector containing a selectable marker (such as a neomycin resistance gene), the vectors being otherwise essentially identical, allows the generation and selection of host cells having desirable properties with respect to expression of the recombinant polypeptide.

Accordingly, in a first aspect the present invention provides a method for producing a transformed eukaryotic host cell which expresses a recombinant polypeptide of interest which method comprises introducing into a eukaryotic host cell:

(a) a first polynucleotide vector which comprises (i) a first nucleotide sequence which encodes a recombinant polypeptide of interest; and (ii) a second nucleotide sequence encoding a selectable marker, which second nucleotide sequence is amplified when the host cell is contacted with a selection agent, and
(b) a second polynucleotide vector having the same nucleotide sequence as the first polynucleotide vector except that the second nucleotide sequence is replaced with a third nucleotide sequence which encodes a different selectable marker;

the first polynucleotide vector and second polynucleotide vector being integrated into the genome of the host cell.

Advantageously and preferably, the first and second polynucleotide vectors are simultaneously introduced into the host cell. In the alternative, the first and the second polynucleotide vectors are introduced into the host cell successively.

Preferably the host cell is a mammalian cell, more preferably a Chinese hamster ovary (CHO) cell.

The selection agent can be any compound or composition which is capable of causing amplification of the second nucleotide sequence when the host cell is contacted with such selection agent. Preferably the second nucleotide sequence encodes a dihydrofolate reductase polypeptide and the selection agent is methotrexate. Preferably the recombinant polypeptide of interest is human erythropoietin.

Typically, the third nucleotide sequence encodes resistance to an antibiotic, such as G-418 or neomycin (i.e. the third nucleotide sequence encodes neomycin phosphotransferase). As will be apparent to those spilled in the art, different antibiotics of the neomycin group may also be used.

Preferably, for all the methods according to the present invention, as disclosed herein, the first nucleotide sequence, the second nucleotide sequence and the third nucleotide sequence are operably linked to a strong promoter, typically a viral promoter such as an SV40 early promoter. Preferably, each of the three nucleotide sequences is independently operable linked to such a strong promoter, preferably to the SV40 early promoter. However, the use of respective polycistronic expression units will also be possible.

Likewise preferably, for all the methods according to the present invention, as disclosed herein, the first nucleotide sequence, the second nucleotide sequence and the third nucleotide sequence are operably linked to further control elements, like a transcription termination control sequence. In a preferred embodiment thereof, such a control element is a sequence encoding a SV40 large T polyadenylation signal. Preferably, such SV40 large T polyadenylation signal sequence includes the known natural occuring intervening sequence.

In a second aspect of the invention, the present invention provides a eukaryotic host cell producible by the method of the first aspect of the invention. Preferably the recombinant polypeptide of interest expressed by the host cell is erythropoietin, typically hEpo. Preferably the host cell is a mammalian cell, more preferably a CHO cell.

In a third aspect the present invention provides a transformed eukaryotic host cell comprising integrated into one or more of its chromosomes;

(a) a first polynucleotide vector which comprises (i) a first nucleotide sequence which encodes a recombinant polypeptide of interest, and (ii) a second nucleotide sequence encoding a selectable marker, which second nucleotide sequence is amplified when the host cell is contacted with a selection agent, and

(b) a second polynucleotide vector having essentially the same nucleotide sequence as the first polynucleotide vector except that the second nucleotide sequence is replaced with a third nucleotide sequence which encodes a different selectable marker.

Preferably the recombinant polypeptide of interest expressed by the host cell is erythropoietin, typically hEpo. Preferably the host cell is a mammalian cell, more preferably a CHO cell.

In a preferred embodiment, the second nucleotide sequence encodes a dihydrofolate reductase polypeptide. Preferably the recombinant polypeptide of interest is erythropoietin, typically hEpo.

In a specific embodiment, the present application discloses a mammalian host cell comprising, integrated, in particular stably intergrated, into one or more of its chromosomes, (a) a polynucleotide sequence which encodes a dihydrofolate reductase polypeptide and erythropoietin, and (b) a polynucleotide sequence, which is distinct from the polynucleotide sequence of (a) and which encodes erythropoietin and a polypeptide which confers resistance to an antibiotic.

Preferably the host cell is a CHO cell. Preferably the antibiotic is G-418, neomycin or a different (but similar) antibiotic of the neomycin group.

In a fourth aspect, the present invention provides a method for producing a recombinant polypeptide of interest which method comprises culturing a host cell according to the second or third aspects of the invention under conditions that permit expression of the first nucleotide sequence. Typically, the host cell is also cultured in the presence of a selection agent that causes amplification of the first nucleotide sequence. Preferably, the second nucleotide sequence encodes a dihydrofolate reductase polypeptide and the selection agent is methotrexate.

In a preferred embodiment, the recombinant polypeptide of interest is human Epo.

Preferably, the recombinant polypeptide is secreted into the culture medium. Typically, the method further comprises recovering the expressed recombinant polypeptide of interest from the culture medium. Alternatively, or additionally, however, the recombinant polypeptide can also be recovered from the transformed host cells.

Moreover, there is described herein a composition comprising glycosylated recombinant human erythropoietin produced by the method of the fourth aspect of the invention.

In a fifth aspect, the present invention provides a composition comprising:

(a) a first polynucleotide vector which comprises (i) a first nucleotide sequence which encodes a recombinant polypeptide of interest, and (ii) a second nucleotide sequence encoding a selectable marker, which second nucleotide sequence is amplified when the host cell is contacted with a selection agent capable of causing amplification of the nucleotide sequence when the host cell is contacted with such selection agent, and

(b) a second polynucleotide vector having essentially the same nucleotide sequence as the first polynucleotide vector except that the second nucleotide sequence is replaced with a third nucleotide sequence which encodes a different selectable marker.

Preferably the recombinant polypeptide of interest is human erythropoietin. Preferably the second nucleotide sequence encodes dihydrofolate reductase. Preferably the third nucleotide sequence encodes neomycin phosphotransferase.

The present invention also provides the use of said composition in a method of producing a eukaryotic host cell which expresses the recombinant polypeptide of interest.

In the context of the present invention, the preferred or particular embodiments as described herein are capable of being combined with each other, thereby resulting in further preferred embodiments thereof.

Detailed description of the invention

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic and biochemical methods (see generally, Sambrook et al., Molecular Cloning: A Laboratory Manual, 2nd ed. (1989) Cold Spring Harbor Laboratory Press, Cold Spring Harbor, N.Y. and Ausubel et al., Short Protocols in Molecular Biology (1999) 4th Ed, John Wiley & Sons, Inc. - and the full version entitled Current Protocols in Molecular Biology, which are incorporated herein by reference) an chemical methods.

Definitions

A eukaryotic host cell as referred to herein may be any cell of eukaryotic origin, whether a primary cell or an established, immortalized cell, which may be adapted for the expression of heterologous polypeptides. Thus, the term, in its broadest embodiment, includes primary cells in culture, tissues and organs in organ culture, tissue implants, whole organisms and established cell lines. In a preferred embodiment, the term refers to cells in culture derived from established cell lines, such as CHO, BHK, COS and HeLa cells.

Expression, as referred to herein, is the production of a polypeptide product from a nucleic acid coding sequence, typically by transcription/translation of the sequence. Expressed polypeptides may be secreted from the host cell or may accumulate therein; preferably, expression according to the present invention includes secretion of the polypeptides from the cells. Expression levels may vary, but are advantageously high; about 1%, preferably 10%, 25% or 50% or even more of the total protein produced by the cell may be the expressed polypeptide of interest.

A "recombinant polypeptide, may be any polypeptide which it is desired to produce by recombinant nucleic acid technology. As used herein, the terms "polypeptide" and "peptide" refer to a polymer in which the monomers are amino acids and are joined together through peptide or disulfide bonds. Polypeptide refers to either a full-length naturally-occurring amino acid chain or a "fragment thereof" or "peptide", such as a selected region of the polypeptide that is of interest, or to an amino acid polymer, or a fragment or peptide thereof, which is partially or wholly non-natural. "Fragment thereof" thus refers to an amino acid sequence that is a portion of a full-length polypeptide, between about 8 and about 500 amino acids in length, preferably about 8 to about 300, more preferably about 8 to about 200 amino acids, and even more preferably about 10 to about 50 or 100 amino acids in length. Where the polypeptide is Epo, fragments thereof are advantageously between about 8 and about 150 amino acids in length, preferably about 15, 20 or 30 to about 50, 100 or 130 amino acids in length. "Peptide" refers to a short amino acid sequence that is 10-40 amino acids long, preferably 10-35 amino acids. Additionally, unnatural amino acids, for example, β-alanine, phenyl glycine and homoarginine may be included. Commonly-encountered amino acids which are not gene-encoded may also be used in the present invention. All of the amino acids used in the present invention may be either the D- or L-optical isomer. The L-isomers are preferred. In addition, other peptidomimetics are also useful, e.g. in linker sequences of polypeptides of the present invention (see Spatola, 1983, in Chemistry and Biochemistry of Amino Acids, Peptides and Proteins, Weinstein, ed., Marcel Dekker, New York, p. 267).

Suitable recombinant polypeptides include proteins of pharmaceutical interest, such as Epo, clotting factors such as factor VIII and factor IX, coagulating agents such as hirudin, hormones, including insulin, human and animal growth hormones, follicle stimulating hormone, luteinizing hormone, other therapeutic proteins such as antibodies and other immunoglobulins, alpha-globin, beta-globin, gamma-globin, granulocyte macrophage-colony stimulating factor, tumor necrosis factor, interleukins, macrophage colony stimulating factor, granulocyte colony stimulating factor, mast cell growth factor, tumor suppressor p53, retinoblastoma, interferons, melanoma associated antigen or B7 and any other proteins whose expression is desired, especially in large quantities.

A "polynucleotide vector" is a nucleic acid vector which may be used to deliver a nucleic acid coding sequence to the genome of a cell, such that it will integrate therein. Integration into the host cell genome leads to stable transformation (as opposed to transient transformation commonly associated with episomal vector maintenance) of the host cell and may be referred to as "stable integration". Polynucleotide vectors, which are described in more detail below, may be plasmids, infectious agents (viruses, virus-like particles, viral nucleic acid), cosmids, phagemids, transposons, packaged DNA or any other form of nucleic acid which is suitable for delivery to cells. Typically, plasmids are employed due to their ease of use, familiarity in the art and freedom from regulatory constraints required for infectious agents such as viruses. "Integration", or "stable integration", refers to the integration of at least part of the nucleic acid sequences of the vector into the genome of the host cell or its maintenance in an artificial chromosomal body; at least the first and second/third nucleotide sequences are preferably integrated into the host cell genome. Advantageously, the entire sequence of the vector is integrated into the host cell genome.

"Transformation" refers herein to the introduction of genetic material into a cell such that the genetic material is expressed within the cell, which is said to be "transformed". For the avoidance of doubt, "transformation" does not refer to the immortalization of cells by oncogenes or oncogenic viruses and the like.

"amplification" refers to the increase in copy number of coding sequences in response to selective pressure. Thus, it is known that, in response to selection using MTX, heterologous dhfr genes are amplified and increase in copy number at the locus in which they are integrated into the host cell genome. Sequences linked to the dhfr genes (i.e., sequences physically proximal thereto) are amplified together with the dhfr genes. This leads to increase in copy number of sequences of interest, and increased expression of gene products encoded thereby.

"Selection agents" are typically agents which place cells under stress or other competitive disadvantage, unless a certain gene product is present In the cell which alleviates the effects of the selection agent. Thus, methotrexate is toxic to cells, but is neutralized by dihydrofolate reductase (DHFR), the gene product of the dhfr gene. Common amplifiable systems include dihydrofolate reductase (DHFR) - methotrexate (MTX), the bacterial xanthine-guanine phosphoribosyl transferase enzyme (that mediates resistance to mycophenolic acid), and amplifying in the presence of a normal functioning gene (such as amplifying glutamine synthetase in the presence of methionine sulfoximine [MSX]. Such systems are amplifiable as such in that the nucleic acid is itself amplified in response to selection pressure applied by cognate agents.

Systems which are not usually regarded as amplifiable as such include, for example antibiotic resistance genes, which encode gene products which confer resistance to antibiotics such as ampicillin or G-418/neomycin.

The vectors according to the invention possess sequences which are similar apart from the selectable markers. The first selectable marker is an amplifiable gene; the second need not be amplifiable. As used herein, "the same" means that the sequences of the vectors, with the exception of the selectable marker sequences, are identical or nearly so. In practice, the two vectors are typically based on the same starting vector, in which the selectable marker gene is replaced. Thus, the remaining sequences will be Identical, save for any spontaneous changes which may occur. Advantageously, the sequences, excepting the marker genes including their respective control regions, are more than 85% identical; advantageously, 90% identical; and preferably at least 95%, 98%, 99% or even completely identical.

A. Polynucleotide vectors

The method of the present invention for producing host cells involves introducing two substantially identical vectors which differ essentially by virtue of one vector comprising a sequence encoding a gene which is amplified when integrated into a host cell chromosome in response to selection, with the other vector comprising a nucleotide sequence encoding another selectable marker, such as an antibiotic resistance gene.

Preferably, the second selectable marker is not amplifiable as such.

The vectors, which as typically circular plasmids, although they may be linear, are generally DNA molecules. The vectors may conveniently include a prokaryotic origin of replication to permit their propagation in bacterial cells such as *E. coli.*

The first vector comprises a first nucleotide sequence which is essentially an expression cassette that is capable of directing/directs expression of a recombinant polypeptide of interest (POI), such as Epo, in a eukaryotic host cell, typically mammalian cell. Accordingly, this first expression cassette comprises the actual coding sequence of the POI operably linked to a control sequence that is capable of providing for the expression of the coding sequence by the host cell. The term "operably linked" means that the components described are in a relationship permitting them to function in their intended manner. A regulatory sequence "operably linked" to a coding sequence is ligated in such a way that expression of the coding sequence is achieved under conditions compatible with the control sequences.

Control sequences operably linked to sequences encoding the POI include promoters/enhancers and other expression regulation signals, including transcriptional terminators. These control sequences may be selected to be compatible with the host cell for which the expression vector is designed to be used in. The term "promoter" is well-known in the art and encompasses nucleic acid regions ranging in size and complexity from minimal promoters to promoters including upstream elements and enhancers.

The promoter is typically selected from promoters which are functional in mammalian cells. The promoter is typically derived from promoter sequences of viral or eukaryotic genes. For example, it may be a promoter derived from the genome of a cell in which expression is to occur. Viral promoters may advantageously be used since they generally direct high levels of transcription. Examples include the SV40 early promoter, and active fragments thereof, the Moloney murine leukemia virus long terminal repeat (MMLV LTR) promoter, the Rous sarcoma virus (RSV) LTR promoter or the human cytomegalovirus (CMV) IE promoter.

Since the replication of vectors is conveniently done in E. coli, an E. coli genetic marker and an E. coli origin of replication are advantageously included. These can be obtained from E. coli plasmids, such as pBR322, Bluescript© vector or a pUC plasmid, e.g. pUCl8 or pUC19, which contain both E. coli replication origin and E. coli genetic marker conferring resistance to antibiotics, such as ampicillin.

It may also be advantageous for the promoters to be inducible so that the levels of expression of the POI can be regulated during the life-time of the cell. Inducible means that the levels of expression obtained using the promoter can be regulated.

The control sequences may be modified, for example by the addition of further transcriptional regulatory elements, for example enhancer sequences, to make the level of transcription directed by the control sequences more responsive to transcriptional modulators. Chimeric promoters may also be used comprising sequence elements from two or more different promoters described above.

In preferred embodiment, the POI is erythropoietin, in particular human Epo. The sequence of human Epo is given in GenBank Accession No. M 11319.1 However, it may be desirable to modify the sequence of human Epo to introduce one or more additional sites for carbohydrate attachment, such as [Asn60]Epo, [Asn125, Ser127]Epo, [Thr125] Epo and [Pro124, Thr125]Epo as described in US Patent No. 5,856,298. The first vector also comprises a second nucleotide sequence which is essentially an expression cassette which when integrated into the host cell genome is amplified when the host cell is contacted with a selection agent that causes Amplification of the nucleotide sequence. Eukaryotic cells, in particular mammalian cells are capable of amplifying certain genes in response to a selective pressure, typically an enzyme inhibitor, resulting in an increase in the numbers of copies of that gene, which in turn increases levels of expression of the gene. The additional gene copies may be maintained intrachromosomally, or in the form of extrachromosomal genetic material such as minute chromosomes (for review see Genes VI, Lewin, 1997, Oxford University Press, Oxford U.K., pp975-978). A well-characterized example is the dihydrofolate reductase gene which is amplified in response to methotrexate (MTX). Other examples include CAD gene (encodes a protein which is involved in the first three steps of UMP synthesis) which is amplified in response to inhibitors of trans-carbamylase, and glutamine synthetase (see WO87/04462) which is amplified in response to methionine sulphoximine.

This second expression cassette, as in the case of the first expression cassette comprises the actually coding sequence of the gene that is capable of being amplified operably linked to a control sequence that is capable of providing for the expression of the coding sequence by the host cell. Suitable control sequences are described above. The control sequences in the first and second nucleotide sequence may be the same or different.

The second vector is the same as the first vector, except that the coding sequence encoding the gene which is amplified in response to selection pressure (e.g. dhfr) is replaced with a selectable marker, typically a nucleotide sequence encoding a polypeptide which provides antibiotic resistance, such as a nucleotide sequence encoding neomycin phosphotransferase. Other antibiotic resistance genes include genes encoding puromycin or hygromycin resistance.

A key feature of the invention is that the two vectors differ only with respect to having either a second or a third nucleotide sequence. Without wishing to be bound by theory, it is believed that the substantial identity between the two vectors facilitates amplification of the first nucleotide sequence encoding the recombinant polypeptide of interest. The third nucleotide sequence encoding a selectable marker serves as a marker for primary selection in culture, whereas the second nucleotide sequence which is amplified in response to selection pressure, causes Amplification of the integrated sequences including the first nucleotide sequence encoding the recombinant polypeptide of interest.

However, it is not necessary for the regions of the vectors outside of the second/third nucleotide sequences to be 100% identical since a small degree of difference may be permitted. For example, the sequence of the two vectors other than the second/third nucleotide sequences should typically be at least 95%, 98% or 99% identical.

It is nonetheless convenient to use vectors that are identical outside of the second/third nucleotide sequences because in practice, the same vector is used as the basis of cloning the first and second vector. For example, a vector lacking a second and third nucleotide sequence but containing a suitable cloning site may be used to generate a cloning vector into which a second or third nucleotide sequence of choice may be inserted.

B. Production of host cells which express a recombinant protein of interest

The first and second polynucleotide vectors may be introduced into a suitable host cells using any suitable technique. For example, uptake of nucleic acids by mammalian cells is enhanced by several known transfection techniques for example electroporation or those including the use of transfection agents. Example of these agents include cationic agents (for example calcium phosphate and DEAE-dextran) and lipofectants (for example lipofectam™ and transfectam™). Typically, the vectors are mixed with the transfection agent to produce a composition. It may be desirable to use an excess of the second vector in relation to the first vector. For example, the first and second vector may be mixed at a ratio of from 1:5 to 1:100 (first:second), more particularly from 1:10 to 1:50.

Preferred host cells are mammalian cells, such as rodent or human cells, and include CHO, BHK, COS and HeLa cells. Particular preferred cells are CHO cells. In a preferred embodiment, the host cells are initially deficient for the gene present in the second nucleotide sequence (e.g. dhfr). CHO. cells deficient in dhfr are described by Urlaub and Chasin, 1980, PNAS 77: 4216-4220 and are available from the ATCC as deposit ATCC CRL-9096. They have been deposited under the Budapest Treaty under deposit designation no. ATCC PTA-3672 at the American Type Culture Collection (ATCC), Rockville, Md. 20852, USA, on August 29, 2001. Examples of immortalized human cell lines which may be used with the DNA constructs and methods of the present invention further include, but are not limited to, HT1080 cells (ATCC CCL 121), HeLa cells and derivatives of HeLa cells (ATCC CCL 2, 2.1 and 2.2), MCF-7 breast cancer cells (ATCC BTH 22), K-562 leukemia cells (ATCC CCL 243), KB carcinoma cells (ATCC CCL 17), 2780AD ovarian carcinoma cells (Van der Blick, A.M. et al., Cancer Res, 48:5927-5932 (1988), Raji cells (ATCC CCL 86), WiDr colon adenocarcinoma cells (ATCC CCL 218), SW620 colon adenocarcinoma cells (ATCC CCL 227), Jurkat cells (ATCC TIB 152), Namalwa cells (ATCC CRL 1432), HL-60 cells (ATCC CCL 240), Daudi cells (ATCC CCL 213), RPMI 8226 cells (ATCC CCL 155), U-937 cells (ATCC CRL 1593), Bowes Melanoma cells (ATCC CRL 9607), WI-38VA13 subline 2R4 cells (ATCC CLL 75.1), and MOLT-4 cells (ATCC CRL 1582), as well as heterohybridoma cells produced by fusion of human cells and cells of another species. Secondary human fibroblast strains, such as WI-38 (ATCC CCL 75) and MRC-5 (ATCC CCL 171) may be used.

Transfections may conveniently be performed in microtitre plates, but other culture vessels may be used. Following transfection, cells are typically cultured in selection medium (i.e. medium that selects for cells that express the selectable marker) to confluence and then in amplification medium containing a selection agent that causes amplification of the second nucleotide sequence, such as MTX. Typically, in the case of MTX, the agent is present in a concentration of about 48 nM ($4.8 \times 10^{-8}$ M).

Clones are then selected, typically grown in the same or similar concentration of selection agent and screened for production of recombinant protein by, for example, ELISA and/or immunofluorescence. The highest producers are then selected, based on the results of the screen.

Ideally, the selected clones are then further tested to determine their growing properties, chromosomal stability, recombinant protein productivity and recombinant protein activity.

Chromosomal stability can be determined for example by looking at cell morphology and/or performing an analysis of DNA content, such Coulter Counter nucleus DNA analysis. Generally, clones which have larger nuclei have a greater than diploid chromosome complement. These clones are likely to be less stable and are generally rejected. Preferred clones are those which have a substantially identical nuclear DNA content to the parent host cell prior to transfection and selection.

Recombinant protein productivity can be tested by SDS-PAGE/Western blot of cells and/or culture supernatant where the protein is secreted (as is the case for Epo). Testing may be conducted at a range of MTX (or other appropriate selection agent). It may be desirable to give consideration to not only the amount of protein but the pattern of protein (especially where multiple isoforms, such as differentially glycosylated isoforms, are produced, as is the case for Epo).

The method of the invention preferably further comprises one or more sub-cloning steps where selected clones are subjected to increasing concentrations of the selection agent which causes amplification of the second nucleotide sequence. In the case of dhfr and MTX, typically initial cloning steps use $10^{-8}$ M concentrations of MTX, increasing up to $10^{-6}$ M concentrations.

Thus, in a further sub-cloning step, cell clones are test cultured in the presence of a range of concentrations of the selection agent (e.g. MTX) and the recombinant protein titre determined. Sub-cloning is then preferably conducted at or above a concentration of the agent where the cell clones do not show a significant increase in protein production compared with lower concentrations. In the case of Epo and CHO cells, this is about 380 nM ($3.8 \times 10^{-7}$ M). In a preferred embodiment, the cells are cultured at progressively higher concentrations of agent up to the highest desired concentration.

Sub-clones may then be further tested to determine their properties as described above. Optionally, further sub-cloning steps, typically with increased concentrations of selection agent (such as MTX), may be performed. In the Examples, a sub-cloning step is performed in the presence of 1.54 μM ($1.54 \times 10^{-6}$ M) MTX. In other cases the MTX concentration may be increased as high as or even higher than 10 μM ($1 \times 10^{-5}$ M).

Preferably, selected host cells of the invention which express Epo, express Epo at a rate of greater than 10 μg/$10^6$ cells/day at an MTX concentration of $7.7 \times 10^{-7}$ M or $1.54 \times 10^6$ M, more preferably greater than 20, 25 or 30 μg/$10^6$ cells/day. It is also preferred than host cells of the invention have a substantially identical nuclear DNA content to the parent host cell prior to transfection (e.g. a CHO dhfr- cell).

It may also be desirable to adapt host cells of the present invention to serum-free media, typically after the final sub-cloning step. This may be achieved by cultivating seeded cells to confluence and replacing the growth medium with serum-free medium or gradually reducing serum concentration to zero over several passages. Preferably the cells are also adapted to growth as a suspension culture.

Selected host cell-lines may be cyropreserved in liquid nitrogen using standard techniques.

C. Protein production

Host cells of the invention may be used to express the recombinant protein of interest, such as rhEpo. Methods for expressing polypeptides using transfected host cells are known in the art. The culture conditions will be selected so as to achieve expression of the polypeptide of interest from the first nucleotide sequence. According to the present invention, it is preferred to use suspension culture methods or roller bottle culture methods. Culturing of host cells for the purpose of expressing the polypeptide of interest may be performed in the presence or absence of the selection agent (i.e. MTX or the appropriate equivalent). Preferably, host cells are cultured in serum-free medium.

The recombinant protein may be recovered from the culture supernatant or a pellet of the cultured cells as appropriate (from the culture supernatant in the case of Epo expression). The recombinant protein is then typically subjected to one or more purification steps (see for example Broudy et al., 1988, Archives of Biochemistry and Biophysics 265: 329-336; Ghanem et al., 1994, Preparative Biochemistry 24(2): 127-142) such as affinity chromatography and/or ion-exchange chromatography.

Preferably in a composition comprising a recombinant polypeptide, particularly Epo, expressed in and purified from a host cell of the invention, the recombinant polypeptide is substantially pure, such as at least 90%, 95% or 99% pure.

The biological activity of the purified protein can be determined *in vitro* and/or *in vivo*. A suitable *in vitro* test is described in Hammerling et al., 1996, J Pharm Biomed Anal 14(11):1455-69, which involves testing for proliferative stimulation of an erythroid cell line. A suitable *in vivo* test is described in Ghanem *et al.,* 1994, supra, which involves determining the incorporation of $^{59}$Fe into red blood cells of polycythemic mice.

In a preferred embodiment, the present invention employs cells cultured by (a) providing a host cell which comprises a nucleotide sequence which encodes the recombinant polypeptide of interest and which directs expression of the recombinant polypeptide of interest in the host cell;(b) providing a serum-free culture medium which comprises (i) water, a plant-derived peptone, an osmolality regulator, a buffer, an energy source, amino acids, a lipid source or precursor, a source of iron, non-ferrous metal ions and one or more vitamins and cofactors; and (ii) does not contain-any-full-length polypeptides; and (c) culturing the host cell in the culture medium under conditions that allow for expression of the recombinant polypeptide of interest.

Preferably the recombinant polypeptide of interest is human erythropoietin. The host cell may be a Chinese hamster ovary (CHO) cell. Such techniques are described in the examples section of this document.

Erythropoietin may be purified from cell culture by (a) removing host cells, cellular constituents and debris from the cell culture medium by centrifugation using a disc stack separator followed by a depth filtration step to obtain a clarified culture medium supernatant; (b) adjusting the conductivity of the supernatant to 5 mS/cm or less, and a pH of between about 7.0 and 8.0; (c) applying the supernatant from step (b) to a column comprising an anion exchange chromatographic medium, washing the column, eluting the rhEpo from the column, and collecting the peak fraction(s) that contain rhEpo; (d) subjecting the combined peak fractions from step (c) to a reverse phase chromatography step using a resin that can be run under medium pressure (< 10 bar) and is resistant to high concentrations of NaOH, the rhEpo being eluted using a linear gradient of an organic solvent; (e) applying one or more fractions eluted in step (d) which contain rhEpo to a column comprising anion exchange chromatographic media, washing the column, and eluting the rhEpo using a linear salt gradient; (f) selecting one or more fractions eluted in step (e) which contain rhEpo based on degree of sialylation of the rhEpo; and (g) subjecting one or more fractions eluted in step (f) which contain rhEpo by one or more size exclusion chromatographic steps using a gel filtration medium to remove potential dimers and higher aggregates; and collecting the eluate containing rhEpo.

Advantageously, the anion exchange medium used in step (c) is the ceramic-based ion exchange medium Q-HyperD F™, obtainable from BioSepra. The polystyrene resin used in step (d) is advantageously Source 30RPC™ (Pharmacia), whilst the anion exchange medium used in step (e) is preferably Pharmacia Q Sepharose High Performance™. The gel filtration medium used in step (g) is preferably Pharmacia Superdex 75 prep grade™.

Such methods are described in the examples section of-this-document.

The present invention is described further with reference to the following examples, which are illustrative only and non-limiting. In particular, the examples relate to preferred embodiments of the present invention.

## EXAMPLES

### *Materials*

Host cell line

Chinese hamster ovary (CHO) dihydrofolate-reductase deficient (ATCC CRL-9096). They have been deposited under the Budapest Treaty under deposit designation no. ATCC PTA-3672 at the American Type Culture Collection (ATCC), Rockville, Md. 20852, USA, on August 29, 2001.

Cell culture Media

| | |
|---|---|
| Cultivation medium: | DMEM supplemented with L-Glutamine, 4mM, 10% FCS, HT (Hypoxanthine, Thymidine), 1x |
| Selection medium: | DMEM supplemented with L-Glutamine, 4mM, dialyzed FCS, 10% and G418, 0.5 mg/ml |
| Amplification medium: | DMEM supplemented with L-Glutamine, 4mM, dialyzed FCS, 10%, G418, 0.5 mg/ml, and MTX (methotrexate), $4.8 \times 10^{-8}$M - $1.54 \times 10^{-6}$M |
| Freezing medium: | DMEM supplemented with L-Glutamine, 4mM, FCS, 10% and DMSO, 10%. |
| Serum-free adaptation medium for recombinant cell-lines: | 1:1 DMEM/Ham's F12, supplemented with: L-Glutamine, 6mM, Soya-peptone/UF, 0.25%, Hybridoma Supplement, 1x, Pluronic-F68, 0.1 %, G418, 0.5 mg/ml, MTX, $1.54 \times 10^{-6}$M |
| Serum-free production medium: | 1:1 DMEM/Ham's F12, supplemented with: Soya-peptone/UF, 0.25%, Hybridoma Supplement, 1x, Lutrol, 0.1 %, MTT, $1.54 \times 10^{-6}$M, Glucose 1 g/l, $NaHCO_3$, 2.5g/l |
| Serum-free freezing medium for recombinant cell-lines: | PBS, PVP-10, 20%, DMSO, 5% |
| | Hybridoma Supplement, 100x Ethanolamine - $2.5 \times 10^{-3}$ M, Ferric-Citrate - $2.5 \times 10^{-2}$ M, L-Ascorbic Acid - $2.0 \times 10^{-3}$ M, Sodium Selenite - $5.0 \times 10^{-6}$ M |

Plasmid constructs

*pEpo/neo*

This plasmid encodes the coding region of Epo and the neomycin resistance gene as two different expression cassettes each under control of SV40 early promoter/terminator sequences. The construction details are provided in Example 1.

*pEpo/dhfr*

This plasmid encodes the coding region of Epo and dhfr as two different expression cassettes each under control of SV40 early promoter/terminator sequences. The construction details are provided in Example 2.

### *Methods In cell culture*

Growth of CHO-dhfr⁻

Dihydrofolate reductase deficient CHO cells (Urlaub et al., 1980, PNAS 77(7):4216-4220) (referred to as CHO dhfr) are cultivated in DMEM cultivation medium with a splitting ratio 1:10 twice a week.

Transfection of CHO cells

$1\text{-}5\text{x}10^4$ cells per $cm^2$ are seeded in $25cm^2$ T-flask bottles or 96-well plates the day before the lipofectin transfection is performed. The corresponding plasmids are mixed in the appropriate ratio, added to the lipofectin reagent (GIBCO/BRL) according to the manufacturer's protocol ($0.5\text{-}1 \mu l/cm^2$). Then the cells are overlaid with the transfection cocktail for four to sixteen hours in serum-free DMEM, before the DNA-containing medium is replaced with cultivation medium. After cultivation for 24 to 48 hours in the serum-containing medium the cells are switched to selection medium. Transfected cell pools are first cultivated in selection medium to confluence and then in amplification medium ($4.8\text{x}10^{-8}$ M MTX) before screening the cell culture supernatants by ELISA for Epo production. Highest producers are determined, the MTX concentration increased two-fold and best producers used for further cultivation.

*Analytical methods*

ELISA detecting Epo in different matrices

**Antibodies**

polyclonal serum: rabbit anti Epo biotinylated (R&D Systems; Catalog # AB-286-NA) monoclonal antibody: mouse anti Epo (Genezyme; Cat. code AE7A5)

**ELISA buffers**

| | |
|---|---|
| Coating buffer: | 8.4 g/l $NaHCO_3$, 4.2 g/l $Na_2CO_3$, pH 9.6-9.8 |
| Washing buffer: | 0.2 g/l KCl, 1.15 g/l $Na_2HPO_4$ x $2H_2O$, 0.2 g/l $KH_2PO_4$, 8 g/l NaCl, 0.1 % Tween 20 |
| Dilution buffer: | 2% PVP (Sigma Cat.No. PVP-4OT) in washing buffer |
| Sample buffer: | 0.5% alpha mono-thio-glycerol in dilution buffer |
| Staining buffer: | 7.3 g/l Citric acid x $2H_2O$, 11.86 g/l $Na_2HPO_4$x$2H_2O$, pH 4.8-5.0 |
| Staining solution: | $100\mu l$ OPD solution/10 ml staining buffer $5\mu l$ $H_2O_2$/10 ml staining buffer |
| OPD stock-solution: | PP: L0017 |

**Method**

The ELISA method used detects Epo in ng/ml concentration ranges, in particular with a detection limit in the range of about 10 ng/ml, starting with 500 ng/ml and eight two-fold dilutions. One monoclonal antibody against the first 26 amino acids of Epo functions as a coating layer, binding Epo. In the next step Epo is specifically bound by the catcher antibody. Detection is arranged through a biotinylated Epo recognizing rabbit antiserum. Visualization is performed by staining with OPD after streptavidin-peroxidase coupling to the plate.

Immunofluorescence

Epo expressing CHO-cells are inoculated with $5\text{x}10^4$ cells/200 $\mu l$ on a cover slip in a 6-well plate and incubated for 24-72 hours. The adherent cells are washed twice with PBS and fixed for 5 minutes with -20°C methanol, then air dried and afterwards again soaked in PBS. Unspecific proteins are saturated by incubation with 20% FCS in PBS for 15 minutes and then anti-Epo is incubated for one hour. The reaction is visualized with anti-mouse IgG FITC conjugated. The fluorescence is detected by confocal microscopy at an excitation wavelength of 488 nm and an emission wavelength of higher then 515 nm.

Nucleus size determination

**Materials**

Coulter Counter® Model ZM (Coulter Electronics Inc.)

Coulter Channelyzer® Model 256
Incubation solution: Citric acid, 0.1M, Triton X 100, 2%
Electrolyte Isoton II (Kat-No.844 8011; Coulter Euro Diagnostic GmbH)

The Coulter Counter quantifies particles in size and number, that are suspended in an electric conductive fluid. The fluid is absorbed by vacuum through a capillary which carries electrodes on both sides. The change of the resistance induces a voltage impulse that can be digitized by the Coulter Channelizer. Nucleus size correlates with DNA content of the cells, so that it is possible to distinguish between a diploid and a polyploid set of chromosomes.

## Method

Approximately $1 \times 10^6$ CHO-cells are washed once in PBS, resuspended in 2 ml incubation solution and left there for 4-5 hours. The following steps are specific for the Coulter Counter.

SDS Polyacrylamide-Electrophoresis and Western blotting

### Materials

| | |
|---|---|
| SDS Gels: | Novex Tris-Glycine 4-20% |
| Sample buffer: | Tris Glycine SDS 2x (Novex LC 2676) |
| Running buffer: | Tris Glycine SDS (Novex LC 2675) |
| Blotting buffer: | $Na_2B_4O_7 \times 10H_2O$, 50 mM, SDS, 0.1 %, methanol, 20% |
| Blotting matrix: | PVDF Immobilon P 0.45$\mu$M Millipore; K8JM8238H |
| Washing buffer: | see ELISA washing buffer |
| Dilution buffer: | 1 % milk powder in washing buffer |
| Detection buffer. | NaCl, 0.1 M |
| | Tris-HCl 0.1 M pH 9.5 |

### Method

Epo containing samples are adjusted to 30 ng/20$\mu$l in 1 x sample buffer with 1% $\alpha$-MTG and applied to the SDS gel. At the end of the run, the proteins are blotted to a PVDF immobilon membrane for two hours and then the Epo is specifically stained with a monoclonal antibody detecting the first 26 amino acids of Epo. Visualization is done with anti mouse IgG conjugated to alkaline phosphatase and NBT/BCIP staining.

Isoelectric focussing

### Materials

| | |
|---|---|
| System: | Multiphor II, Amersham Biosciences |
| IPG Gels: | pH 2.5 -7 |
| Reswelling buffer: | 9 g urea, 0.5 g CHAPS, 0.04 g DTE, 0.5 ml resolytes (pH 3.5-10) 10 $\mu$l bromphenol-blue (0.1-%), adjust to 25 ml with $H_2O$ |
| Sample buffer: | IPG sample buffer pH 3-10, 25$\mu$l in 625 $\mu$l $H_2O$ |
| Blotting buffer: | 2.93 g Glycine, 5.81 g Tris, 20 ml methanol, 0.375 g SDS adjust to 1000 ml with $H_2O$ |
| Blotting matrix: | PVDF Immobilon P 0.45 $\mu$M Millipore; K8JM8238H |
| Washing buffer: | see ELISA washing buffer |
| Dilution buffer: | 1 % milk powder in washing buffer |
| Detection buffer: | NaCl, 0.1 M, Tris-HCl 0.1 M pH 9.5 |

**Method**

Epo containing samples are adjusted to 500-1000 ng/50 $\mu$l, desalted, diluted 1:1 in sample buffer and applied to the reswollen IPG gel. Running conditions are first one minute 300V, then linear increase to 3500V and at the end 1 hour at 3500V. During the whole focussing process a limit of 10 mA and 10W is set. Afterwards the proteins are blotted to a PVDF Immobilon membrane by diffusion overnight or by electroblot and then Epo is stained specifically with a monoclonal antibody detecting the first 26 amino acids of Epo. Visualization is done with anti mouse IgG AP conjugated and NBT/BCIP staining.

Determination of the DNA content

The DNA content of recombinant cell-lines is compared with the CHO-dhfr host cell-line by FACS analysis.

**Materials**

|  |  |
|---|---|
| Washing-Buffer: | 0.1 M Tris-HCl, pH 7.4, 2 mM MgCl$_2$, 0.1% Triton X100 |
| Staining Buffer: | 0.3 $\mu$g/ml DAPI (Hoechst) in washing buffer |

**Method**

$5 \times 10^5$ cells are washed in PBS and fixed with ice cold 70% ethanol. Afterwards the cells are washed twice with washing buffer and then incubated in staining buffer for 30 minutes at RT. The DNA content is measured with the FACS Vantage (Becton and Dickinson) at 359 nm excitation and 450 nm emission.

_In-vitro_ specificity test

The human erythroleukemic cell-line TF-1 (German Collection of Microorganisms and Cell Cultures) is growth-dependent on IL3 or hGM-CSF. These cytokines display synergistic effects on proliferation, while Epo can maintain the viability for some time. The cells are routinely grown in GM-CSF and Epo containing cultivation medium.

**Test Supplements**

|  |  |
|---|---|
| Cultivation medium: | RPMI 1640, supplemented with 4mM Gln, 10% FCS, 20$\mu$g/ml transferrin, 10$\mu$M beta- mercapto-ethanol, 12ng/ml rhGM-CSF, 3U/ml rh Epo, |
| Test medium: | RPMI 1640, supplemented with 4mM Gln, 100$\mu$g/ml transferrin, 2mg/ml BSA |

**Methods**

The functionality test is performed as a MTT viability test in 96-well plates (Hammerling et al., 1996, J Pharm Biomed Anal 14(11):1455-69). Samples are diluted 1:2 fold eight times, starting with 100 ng Epo per ml in test medium. 50 $\mu$l of each sample dilution, standard dilution or blank are transferred to the 96-well testing plate. TF-1 cells are washed three times with cold PBS and adapted to $2 \times 10^5$ cells per ml in test medium. Each well of 96-well test plate is overlayed with 50 $\mu$l of the cell suspension and these cells are left for 72 hours in the CO$_2$ incubator. Afterwards 10 $\mu$l MTT solution (6 mg/ml in PBS) are added and incubated at 37°C for 4 hours. The dye is dissolved with 100 $\mu$l SDS/HCl (10% SDS in 0.1 M HCl) for another 4 hours in the dark and the Epo dependent viability is photomerically determined at 550/690 nm.

**Example 1 - Construction of plasmid Epo/neo**

**1. Construction of p2-neo**

**1.1 Preparation of the vector fragment from pSV2neo containing the SV40 early**

**promoter**

The basis of the vector construction is the pBR322 plasmid backbone contained in pSV2neo. The smaller *Eco*RI - *Pvu*II restriction fragment includes this pBR322 backbone and the neighboring *Pvu*II - *Hin*dIII fragment from SV40 bears the relevant fragment of the SV40 early promoter.

Plasmid pSV2neo (ATCC 37149) is cut with the restriction enzymes *Eco*RI and *Hin*dIII. The two resulting fragments has a sizes of 3092 bp and 2637 bp. The 2637 bp fragment consists of an EcoRI - PvuI restriction fragment including a pBR322 backbone and a neighboring *Pvu*II - *Hin*dIII fragment which contains a fragment of the SV40 early promoter. The 2637 bp is prepared and purified via gel electrophoresis.

**1.2 Preparation of the neomycin resistance gene**

The neo gene is taken from the transposon Tn5 of pSV2neo. It is amplified as a fragment containing solely the coding region of the gene. As part of the cloning strategy, recognition sites for restriction endonucleases are introduced at both ends. A *Hin*dIII site is built in the upstream amplification primer, an *Eco*RI and a Spel site in the downstream primer. The amplified region corresponds to nucleotides 2846 to 1938 in the sequence of pSV2neo (Genbank Accession No. U02434). The oligonucleotides are designed as follows:

| | |
|---|---|
| *Oligo 2004-01:* | length: 38mer |
| 5'- ggg gga agc ttg ttg gga agc cct gca aag taa act gg - 3' | SEQ. ID. No.1 |
| 5' *Hin*dIII: aagctt-**g** (= pos. 2846 in pSV2neo) ttgggaagccctg | SEQ. ID. No. 2 |
| | |
| *Oligo 2004-02:* | length: 42mer |
| 5'- ggg gaa ttc <u>act agt</u> gag tcc cgc tca gaa gaa ctc gtc aag - 3' | SEQ. ID. No. 3 |
| 5' *Eco*RI/*Spe*I: | |
| gaa ttc <u>actagt</u> - **g** (= pos. 1938 in pSV2neo) agtcccgctcagaa | SEQ. ID. No. 4 |

The amplification product of primers 2004-01 and 2004-02 is prepared by PCR using *Pwo* polymerase (Roche Diagnostics). The parameters for the process are: 20 ng of pSV2neo, 10 pmol of each primer, 10 mmol dNTPs, 2.5 U *Pwo* polymerase in the supplied buffer to a total volume of 50 μl; temperature profile: 5 min 95°C, 35 times (30 sec 95 °C, 20 sec 65 °C, 90 sec 72 °C), 3 min 72 °C, cooling at 4 °C until further use.

The resulting DNA fragment of 935 bp is purified by DNA isolation columns (Mini, Wizard Promega GmbH), digested with *Eco*RI and *Hin*dIII, and purified via an agarose gel and eluted using Spin Columns (Supelco).

**1.3 Construction of p1-neo**

The amplified *Eco*RI-*Hin*dIII neo gene fragment is ligated to the *Eco*RI-*Hin*dIII vector fragment from pSV2-neo using Ligation Express (Clontech) and transformed into an *E.coli* host (*E.coli* SURE (Stratagene)). Transformants are selected by growth on LB medium supplemented with 50 mg/l ampicillin.

Plasmid DNA is isolated from clones and checked by restriction analysis using EcoRI plus *Nco*I (3 fragments of 2527 bp, 780 bp and 251 bp, respectively). Plasmid DNAs showing the expected fragments are further checked by sequencing relevant parts of the constructs. A plasmid DNA containing a verified SV40early promoter and neomycin resistance gene is designated as p1-neo.

**1.4 Preparation of the SV40 termination region SV40LTpolyA/IVS**

PCR primers are designed to amplify a fragment (nucleotides 751 to 1598) of the SV40 termination region present in pSV2neo. The upstream primer also contains a restriction site for *Spe*I. In addition to the *Bam*HI site already included at position 751 of pSV2-neo an *Eco*RI site is introduced into the downstream primer separated by a 6 nucleotide spacing region from *Bam*HI. The sequences of the two primers are as follows:

*Oligo 2004_05:*      length: 40mer

5'- ggg <u>gac</u> <u>tag</u> ttt gtg aag gaa cct tac ttc tgt ggt gtg a - 3'      SEQ. ID. No. 5

*5' Spe*l*:* <u>actag</u> **-t** (= pos. 1598 in pSV2neo) ttgtgaagga      SEQ. ID. No. 6

*Oligo 2004-06:*      length: 46mer

5'- ggg gga <u>att</u> ggg agg ggg <u>atc</u> cag aca tga taa gat aca ttg atg a - 3'      SEQ. ID. No. 7

5' *Eco*RI/*Bam*HI*:* <u>gaattc</u> - **g** (= pos. 751 in pSV2neo)<u>gatcc</u> agacatgataag      SEQ. ID. No. 8

The amplification product of primers 2004-05 + 2004-06 is prepared by PCR using *Pwo* polymerase (Roche Diagnostics) as described above. The resulting DNA fragment of 873 bp is purified using DNA isolation columns, digested with EcoR*I* and *Spe*l and gel-purified.

**1.5 Preparation of p2-neo**

p1-neo plasmid DNA is digested using *Eco*RI + *Spe*l. The resulting linearized fragment is purified, ligated with the amplified fragment containing SV40LTpolyA/IVS and transformed into an *E.coli* host. Transformants are selected by growth on LB medium supplemented with 50 mg/l ampicillin.

Plasmid DNA is isolated from clones and checked by restriction analysis using *Eco*RI (1 fragment of 4411 bp) and Ncol (2 fragments of size 3631 bp and 780 bp) and *Sph*l (3 fragments of size 3499 bp, 840 bp and 72 bp). Plasmid DNAs showing the expected fragments are further checked by sequencing relevant parts of the constructs. A plasmid DNA containing a verified SV40LTpolyA/IVS is designated as p2-neo.

## 2. Construction of plasmid p3

### 2.1 Preparation of the SV40 early promoter fragment

Plasmid pSV2neo is used as a source for the SV40 early promoter fragment. The fragment size is almost identical to the one used in constructing the p2 plasmids. However the ends of the fragment are modified to introduce recognition sites for *Bam*HI and *Not*l. The oligonucleotide primers used to amplify the promoter are designed as follows:

*Oligo 2004-07:*      Length: 38mer

5'- ggg <u>ggg</u> <u>atc</u> <u>c</u>tg tgg aat gtg tgt cag tta ggg tgt gg - 3'      SEQ. ID. No. 9

5' *Bam*HI: <u>ggatcc</u> - **t** (= pos. 3435 in pSV2neo) gtggaat      SEQ. ID. No. 10

*Oligo 2004-08:*      Length: 46mer

5'- ggg <u>ggc</u> <u>ggc</u> <u>cgc</u> agc ttt ttg caa aag cct agg cct cca aaa aag c - 3'      SEQ. ID. No. 11

5' Notl: <u>gcggccgc</u> - **a** (= pos. 3093 in pSV2neo) gctttttgcaaaag      SEQ. ID. No. 12

The amplification product of primers 2004-07 + 2004-08 is prepared by PCR using *Pwo* polymerase (Roche Diagnostics), as described above. The resulting DNA fragment of 365 bp is purified using DNA isolation columns, digested with BamHI and *Not*l, and gel-purified.

### 2.2 Preparation of the pBluescript vector part

pBluescript II SK+ DNA is sequentially restricted using *Bam*HI and *Not*l, respectively. The DNA is dephosphorylated using alkaline phosphatase. The *Bam*HI/*Not*l fragment is purified from the small fragment via agarose gel electrophoresis prior to ligation.

### 2.3 Preparation and verification of plasmid p3

The amplified *Bam*HI-*Not*l fragment containing the SV40 early promoter is ligated into the prepared pBluescript II SK+ vector using T4 DNA Ligase (Promega GmbH). Plasmid DNA from *E.coli* SURE (Stratagene) transformants is isolated and purified from colonies on LB medium supplemented with 100 mg/l ampicillin.

Resulting DNAs are checked by restriction analysis using *Eco*RI plus Ncol (2 fragments of size 3039 bp and 253 bp).

Two plasmid DNAs showing the expected fragments are further checked by sequencing. Both strands of the SV40 early promoter are sequenced so that each position could be verified. The plasmid is designated as p3.

## 3. Isolation of human Epo cDNA

### 3.1 Isolation of total RNA with TRIZol® Reagent

TRIzol® reagent, used for isolation of Epo RNA from human kidney tissues (obtained from the Lainzer Krankenhaus hospital) is a mono-phasic solution of phenol and guanidine isothiocyanate. During cell lysis guanidine isothiocyanate forms a water-soluble complex with RNA while cells are disrupted. Addition of chloroform, followed by centrifugation, separates the solution into an aqueous, RNA containing and an organic phase. After separation of the aqueous phase the RNA is precipitated with isopropyl alcohol, washed with ethanol, air-dried and resuspended in RNAse free water.

Human kidney tissue fragments are cut into small pieces, forced through a 100 $\mu$m cell strainer, centrifuged (179xg/10 min) and the resulting pellet is washed three times with PBS. Then the pellet is resuspended in PBS, aliqoted in sterile tubes, frozen to -196°C and stored at -80°C until further use.

The frozen tissue is lysed by addition of 1 ml TRIzol® reagent, homogenized and incubated at 15-30°C for 5 minutes to ensure complete dissociation. After addition of 200 $\mu$l chloroform, shaking the tube and incubation for 2-3 minutes at 15-30°C the tube is centrifuged at 12000xg for 10 minutes. Following centrifugation, the upper aqueous phase is carefully transferred to a fresh tube mixed with 500 $\mu$l isopropyl alcohol and incubated at 15-30°C for 10 min. The precipitated RNA is centrifuged (12000xg, 10 min), the pellet washed with ethanol, centrifuged again, airdried and dissolved in RNAse free DEPC water. Total RNA content is measured photometrically at 260 nm.

$$1 \text{ OD}_{260nm} = 40 \text{ μg RNA/ml}$$

By evaluating the ratio of $OD_{260nm}$ and $OD_{280nm}$ (maximum absorbance of proteins) one can estimate the purity of the RNA isolation. It should range between 1.6 and 1.8.

### 3.2 *mRNA isolation with Dynabeads Oligo (dT)25*

Dynabeads Oligo $(dT)_{25}$ mRNA DIRECT kit employs hybridization of the polyadenosine tail RNA of eukaryotic mRNA to supermagnetic, polystyrene particles containing 25 nucleotide long chains of deoxy-thymidylate covalently attached to their surface. mRNA bound to the magnetic beads can be separated using a Dynal magnetic particle concentrator (Dynal MPC®).

> *Washing buffer* Tris-HCl 10 mM, pH 8,0; LiCl 0,15 mM; EDTA 1 mM
> *2 x Binding buffer* Tris-HCl 20 mM, pH 7,5; LiCl 1 mM; EDTA 2 mM

For 10 $\mu$g of total RNA, 100 $\mu$l Dynabeads oligo $(dT)_{25}$ are separated in the Dynal MPC® and washed twice with 2x washing buffer. Meanwhile total RNA is adjusted to a volume of 200 $\mu$l with 1x washing buffer and denatured by incubation at 65°C for 4 minutes. Then the RNA is mixed with the beads, incubated at room temperature for 5 minutes and separated in the Dynal MPC®. The beads are washed twice with 1x washing buffer. The polyadenylated RNA is eluted from the Dynalbeads Oligo $(dT)_{25}$ by incubation with elution buffer (2 x 10 $\mu$l) for 4 minutes at 65°C. Dynabeads are separated in the Dynal MPC® and the supernatant is immediately transferred to a new RNAse free microcentrifuge tube. The eluate is used directly for reverse transcription.

### 3.3 Reverse Transcription

The specific primer for Epo is denatured by 4 min incubation at 80°C and the mRNA is denatured by 5 min incubation at 65°C. The following components are added to a sterile 1.5 ml microcentrifuge tube on ice:

| Reagent | Final concentration |
| --- | --- |
| mRNA | 10 $\mu$l |
| MMLV (200 U/$\mu$l) | 0,25 $\mu$l |
| Boehringer PCR buffer (10 x) | 2$\mu$l |

(continued)

| Reagent | Final concentration |
|---|---|
| dNTPs (10 mM) | 2$\mu$l |
| MgCl$_2$ (50 mM) | 1 $\mu$l |
| Epo for (100 pmol/$\mu$l) | 1 $\mu$l |
| DTT (0,1 M) | 0,25 $\mu$l |
| RNAse inhibitor (40 U/$\mu$l) | 0,25 $\mu$l |
| H$_2$O | 3,25 $\mu$l |

| | | |
|---|---|---|
| Incubation: | 60 min | 37°C |
| Inactivation: | 5 min | 100°C |

### 3.4 Polymerase chain reaction

The following components are added to a sterile 1.5 ml microcentrifuge tube at 4°C. PCR conditions are listed below.

| Reagent | Epo |
|---|---|
| Template | cDNA Epo 5 $\mu$l |
| polymerase | Vent 1 U (0,5$\mu$l) |
| polymerase buffer (10x) | Vent buffer 1 x (1 0$\mu$l) |
| dNTPs (10 mM) | 200 $\mu$M (2$\mu$l) |
| MgCl$_2$ (50 mM) | / |
| Primer for (10 pM) | 30pM (3$\mu$l) |
| Primer back (10 pM) | 30 pM (3$\mu$l) |
| DMSO | / |
| H$_2$O | 76,5 $\mu$l |

| PCR cycle | |
|---|---|
| 1 Denaturation | 95°C 2 min |
| 2 Denaturation | 94°C 45 sec |
| 3 Primer annealing | 58°C 30 sec |
| 4 extension | 72°C 1 min |
| 5. finish extension | 72°C 10 min |
| 6. cycles | 30 |

The PCR amplification products are analyzed by agarose gel electrophoresis.

### 3.5 Agarose gel electrophoresis

| | |
|---|---|
| 6 x BX buffer | bromphenol blue 0,25 %; xylene cyanol 0,25 %, glycerol 30 % |
| TAE buffer | Tris base 242g; glacial acetic acid 57,1 ml; EDTA (0,5 M, pH 8,0) 100 ml; adjusted to 1000 ml H$_2$O |
| Lambda-Marker III | 10 $\mu$g bacteriophage Lambda-wildtype-Dann (2,5 $\mu$l Hind III + 2,5 $\mu$l Eco R I + 20 $\mu$l buffer R (Fermentas) filled up with H$_2$O ad 200 $\mu$l; 1 h at 37°C digested, 20 min at 65 C inactivated; supplemented with 40 $\mu$l BX-loading buffer) |

1 g agarose and 99g 1xTAE buffer are melted in the microwave oven, cooled down to approximately 60°C and supplemented with 3 $\mu$l of ethidium bromide stock solution (10 mg/ml). Gels are run in 1xTAE buffer at 100-300 V for approximately 30 min, depending on the length of the DNA fragments to be separated. Each lane contains 10 $\mu$l sample mixed with 2 $\mu$l 6 x BX buffer. Identification of DNA fragments is based on comparison with a Lamba/Hind III digest molecular weight standard.

### 3.6 Preparation of PCR products and vectors for ligation

*3.6.1 Restriction of Vector DNA and insert for sticky end cloning*

10u of restriction enzyme and appropriate restriction buffers are mixed with 1$\mu$g vector DNA and insert according to manufacturers instructions. The mixture is incubated at 37°C (30°C for Smal) between 30 and 60 min, depending on the enzymes used, vector and insert. Then the enzyme is inactivated by heating up to 65°C for 10 min and the reaction mixture is analyzed by agarose gel electrophoresis.

*3.6.2 Ligation*

*pIRESneoSV40 vector*

pIRES*neo* vector (Clontech laboratories) contains the internal ribosome entry site (IRES) of the encephalomyocarditis virus (ECMV), which permits the translation of two open reading frames from one messenger RNA. The expression cassette of pIRES*neo* contains the human cytomegalovirus (CMV) major immediate early promoter/enhancer followed by a multiple cloning site (MCS), the ECMV IRES followed by the neomycin phosphotransferase gene and the polyadenylation signal of the bovine growth hormone. In this vector the CMV promoter is replaced by the SV40 early promoter.

Vector and PCR product are ligated with T4 DNA ligase. For optimal ligation approximately 20 ng vector and 200 ng insert (depending on the length) are used in a molar ratio of about 1:10 and mixed with following reagents in a total volume of 10 $\mu$l H$_2$O. The incubation is performed overnight at 15°C and 3 h at RT. Then the ligase is heat-inactivated by incubation at 65°C for 10 minutes.

| Reagent | Final amount |
|---|---|
| Vector (pIRESneoSV40) | ~ 20 ng |
| Insert (Epo) | ~ 200 ng |
| T4 DNA. Ligase | 1U (1 $\mu$l) |
| Buffer (5x) | 1x (2 $\mu$l) |
| H$_2$O | ad 10 $\mu$l |

*3.6.3 Bacteria and culture media*

| | |
|---|---|
| JM109 | (Promega, USA) |
| LB-medium | peptone from casein 10 g; yeast extract 5 g; NaCl 10 g, adjusted to 1000 ml with H$_2$O and set to pH 7,0 with 5M NaOH |
| LB agar | 15 g agar in 1000 ml LB-medium |
| LB-Amp | 100 $\mu$l ampicillin (100 mg/ml) in 1000 ml LB-medium |
| SOC medium | bacto tryptone 20 g; yeast extract 5 g; NaCl 10 mM; KCl 3 mM; MgCl$_2$ 10 mM; glucose 20 mM, MgSO$_4$ 10 mM |

*3.6.4 Transformation using CaCl$_2$*

Preparation of competent bacteria (JM109)

10 ml of LB medium is inoculated with *E.coli* (JM1 09) and grown overnight at 37°C. 4 ml bacterial culture is diluted 1:100 in LB medium and grown until having reached OD$_{260nm}$ of 0.8. Bacteria are centrifuged at 4500 rpm for 10 min at 4°C and the cell pellet is resuspended in 10 ml 0.1 M CaCl$_2$ (4°C)/50 ml bacterial suspension used. The cells are centrifuged, the pellet is resuspended in 2 ml 0.1 M CaCl$_2$ and aliquoted to a total volume of 100 μl, frozen in liquid nitrogen and stored at -80°C.

Transformation

In pre-chilled 17x100 mm polypropylene culture tubes 5-10 ng plasmid DNA is added to JM109 competent bacteria, gently mixed and put on ice for 30 min. Then the cells are heat-shocked for 45 seconds in a waterbath at exactly 42°C without shaking and immediately placed on ice for 2 minutes. Then 900 μl SOC medium is added to the tube and incubated for 30 min at 37°C before plating 100 μl of bacteria suspension on LB-Amp plates.

3.6.5 Screening and establishing glycerin cultures

Ampicillin resistant colonies are screened for the inserted DNA fragment by PCR technique. Portions of ampicillin resistant colonies are mixed with the PCR reaction mixture and with specific primers against the cloned DNA fragment (see below). Positive colonies show PCR-amplified DNA bands in agarose gel electrophoresis. These colonies are then propagated in LB-Amp medium for further analysis and plasmid purification. For further use and storage 1 ml of desired bacteria culture is mixed with 500 μl glycerin (87 %) and stored at -80°C.

3.6.6 Wizard® Plus SV Minipreps DNA Purification System

| | |
|---|---|
| *Cell resuspension solution* | Tris-HCl 50 mM, pH7.5; EDTA 10 mM, RNase A 100 μg/ml |
| *Cell lysis solution* | NaOH 0.2 M, SDS 1 % |
| *Neutralization solution* | guanidine hydrochloride 4.09M, potassium acetate 0.759M; glacial acetic acid 2.12M, pH 4.2 |
| *Column wash solution* | potassium acetate 60 mM, Tris-HCl 10 mM, pH 7.5; ethanol 60% |

2-3 ml LB-Amp medium are inoculated with a single colony and incubated at 37°C over night. The solution is centrifuged (12000xg, 5 min) and the resulting pellet is thoroughly resuspended in 250 μl resuspension solution and then 250 μl of cell lysis solution, mixed by inverting the tubes 4 times and incubated at RT for 1-5 min. Thereafter 10 μl of alkaline protease solution (incubated at RT for 5 min) and 350 μl neutralization solution ware added. The tube is immediately mixed by inverting it 4 times and the bacterial lysate is centrifuged at 12000xg for 10 min at RT. The cleared lysate is transferred to Spin Columns and centrifuged (12000xg, 5 min) and the column is washed twice with washing solution (750 μl/250 μl). The DNA is eluted with 100 μl nuclease-free water.

3.6.7 Sequencing of plasmids

The inserted sequences are sequenced by IBL (Gerasdorf, Austria) and by GenXpress (Maria Wörth, Austria) with specific primers. Oligonucleotide primers for the amplification of Epo and SV40early-promoter-and for sequence analysis are listed below.

| Oligonucleotide primer | Sequence |
|---|---|
| *SV40early promoter* | |
| SV40 early ClaI for | 5'-aga tcg atc aag ctt ttt gca aaa gcc tag-3' SEQ. ID. No. 13 |
| SV40 early NruI back | 5'-agt cgc gag cgc agc acc atg gcc tg-3' SEQ. ID. No. 14 |
| SV40 early 281 back | 5'-gcc cag ttc cgc cca ttc-3' SEQ. ID. No.15 |
| *Epo* | |
| Epo BamHI for | 5'-tag gat cct cat ctg tcc cct gtc ctg c-3' SEQ. ID. No. 16 |
| Epo EcoRI back | 5'-tag aat tcc gcc atg ggg gtg cac gaa tgt cc-3' SEQ. ID. No. 17 |
| Epo 221 for | 5'-taa ctt tgg tgt ctg gga-3' SEQ. ID. No. 18 |
| Epo 204 back | 5'-tcc cag aca cca aag tt-3' SEQ. ID. No.19 |

(continued)

| pIRESneo | | |
|---|---|---|
| | pIRESneo 181 back | 5'-tta ggg tta ggc gtt ttg cg-3' SEQ. ID. No.20 |
| | pIRESneo 1016 for | 5'-act cac ccc aac agc cg-3' SEQ. ID. No.21 |
| | pIRESneo 2786 for | 5'-ggcc aaa caa cag atg gct-3' SEQ. ID. No.22 |
| | pIRES-200 back | 5'-tgg aaa gag tca aat ggc-3' SEQ. ID. No.23 |

## 4. Construction of plasmid p5

### 4.1 Preparation of the Epo gene fragment

The structural gene for Epo (human erythropoietin) is amplified by PCR using pSVGPIRNEO as a template DNA. The sequence of Epo is given in GenBank Accession No. M 11319.1. Recognition sites for *Not*I and *Ksp*I are introduced into the upstream and downstream primer, respectively. The primers are designed as follows:

Oligo 2004-09:          Length: 45mer
5'- ggg ggc ggc cgc atg ggg gtg cac gaa tgt cct gcc tgg ctg tgg - 3'      SEQ. ID. No. 24
5' *Not*I: gcggccgc a(= pos. 665 in PSVGPIRNEO) tgggggtg      SEQ. ID. No.25

Oligo 2004-10:          Length: 44mer
5'- ggg gcc gcg gtc atc tgt ccc ctg tcc tgc agg cct ccc ctg tg - 3'      SEQ. ID. No.26
5' *Ksp*I: ccgcgg - **t** (= pos. 1246 in PSVGPIRNEO) catctgtcccct      SEQ. ID. No.27

The amplification product of primers 2004-09 +2004-10 is prepared by PCR using *Pwo* polymerase (Roche Diagnostics), as described above. The resulting DNA fragment of 604 bp is purified using DNA isolation columns, digested with *Ksp*I and *Not*I, and gel-purified. The resulting 592 bp *Ksp*I/*Not*I Fragment is used in the triple ligation described below.

### 4.2 Preparation of the termination region SV40LTpolyA/IVS

The termination region of SV40LTpolyA/IVS is recloned from pSV2neo by PCR in a similar manner to that described above in section 1.4 for the construction of p2-neo except that the primers are designed with different restriction endonuclease recognition sites: the site for *Ksp*I (=*Sac*II) is included into the upstream primer and the sites for Sad and EcoRI into the downstream primer.

Oligo 2004-11:          length: 42mer
5'- ggg gcc gcg gtt tgt gaa gga acc tta ctt ctg tgg tgt gac - 3'      SEQ. ID. No. 28
5' *Ksp*I: ccgcgg - t (= pos. 1598 in pSV2neo) ttgtgaaggaa      SEQ. ID. No. 29

Oligo 2004-12:          length: 46mer
5'- ggg gga gct cga att cga tcc aga cat gat aag ata cat tga g - 3'      SEQ. ID. No. 30
5' *Sac*I/*Eco*RI: gagctc gaattc - **g** (= pos. **752** in pSV2neo) atccagacatg      SEQ. ID. No. 31

The amplification product of primers 2004-11 + 2004-12 is prepared by PCR using *Pwo* polymerase (Roche Diagnostics), as described above. The resulting DNA fragment of 873 bp is purified using DNA isolation columns and digested with *Ksp*I and Sad. The resulting DNA fragment of 858 bp is then gel-purified.

### 4.3 Preparation of the p3 vector part

p3 plasmid DNA is sequentially digested using *Not*I and *Sac*I, respectively. The DNA is treated with alkaline phosphatase and the vector fragment is gel-purified.

### 4.4 Triple ligation and isolation of plasmid p5

The *Not*I/*Sac*I vector part of plasmid p3, the *Ksp*I/*Not*I Epo gene and the *Ksp*I/*Sac*I termination region SV40LTpolyA/IVS are ligated in one ligation reaction (Ligation Express, Clontech). Transformants of are selected on LB medium supplemented with 100 mg/l ampicillin.

Positive transformants containing both fragments inserted are screened by colony hybridization using both amplified fragments 2004-09/2004-10 and 2004-11/2004-12, as labeled probes. Ten clones which gave a positive hybridization signal with both probes are chosen for a "midi" scale plasmid preparation (Qiagen).

Restriction analysis is performed using the enzymes *Bam*HI (1 fragment 4723 bp), *Eco*RI (2 fragments, 2913, 1810 bp) and *Pvu*II (4 fragments 2513, 1204, 903, 103 bp). Two clones showing the correct restriction fragments are selected and checked by sequencing. The whole cassette cloned into pBluescript II SK+ is sequenced and compared to the expected nucleotide sequence. Every single nucleotide could be successfully verified. The plasmids are designated p5.

*5. Construction of pEpo/neo*

**5.1 Construction of pEpo/neo 12-1**

p5 plasmid DNA is digested with *Bam*HI and *Eco*RI and the resulting 1792 bp fragment representing the cassette of SV40promoter-Epogene-SV40terminator is gel-purified. Plasmid p2-neo is also digested with *Bam*HI and *Eco*RI and the linearized vector gel-purified. Additionally the DNA is dephosphorylated using alkaline phosphatase and purified with Amicon Micropure enzyme removers.

Both fragments, the 4411 bp p2-neo vector and the 1792 bp cassette from p5, are ligated (Ligation Express, Clontech) and transformed into *E.coli* SURE. Plasmid DNA is isolated from various transformants grown on LB medium supplemented with 70 mg/l ampicillin and analyzed by digestion using restriction endonucleases *Pvu*II, *Eco*RI and *Nco*I.

A clone showing the expected fragments (*Eco*RI: 6191 bp, *Nco*I: 4085, 1326 and 780 bp, *Pvu*II: 3273, 2130, 685 and 103 bp) is selected and designated as pEpo/ neo-12.

For additional purification the DNA is retransformed into *E.coli* SURE (see above) and plasmid DNA prepared using a "Midi-prep" procedure (Qiagen) from a culture inoculated by a single colony (pEpo/neo-12-1). Restriction analysis is performed using the following enzymes: *Bam*HI, *Hind*II, *Eco*RI, *Nco*I, *Not*I, *Pst*I, *Spe*I, *Sph*I, *Pvu*II, *Nar*I. The expected fragments and sizes could be found, verifying the clone as a correct pEpo/neo clone.

**5.2 The final construction of pEpo/neo**

The upstream region of the Epo gene in pEpo/neo-12-1 is changed at position minus-3 from the start ATG. An additional nucleotide A is introduced to result in the purine base G at position -3 from start ATG. A purine at that position may improve the expression level of the gene. For that purpose the Epo gene is reamplified using an adapted upstream primer 2004-09-a:

> *Oligo* 2004-09-a: length: 46mer
>
> 5'- gggggcggccgcaatgggggtgcacgaatgtcctgcctggctgtgg - 3'     SEQ. ID. No. 32

The amplification product of primers 2004-09_a + 2004-10 is prepared by PCR using *Pwo* polymerase (Roche Diagnostics), as described above. The resulting DNA fragment of 605 bp is purified using DNA isolation columns and digested using *Ksp*I and *Not*I. The resulting DNA fragment of 593 bp is then gel-purified.

pEpo/neo-12-1 plasmid DNA is digested with *Ksp*I and *Not*I, respectively, to remove the Epo gene. The 5599 bp fragment is then gel-purified. Both prepared DNAs are ligated to each other (Ligation Express, Clontech). Plasmid DNA from transformants is isolated and purified from colonies on LB medium supplemented with 70 mg/l ampicillin. DNAs are analyzed by restriction using *Nco*I in a first screening.

A positive clone is selected to isolate DNA using a "Midi prep" procedure (Qiagen). An extended restriction analysis is performed using BamHI, *Hind*III, *Eco*RI, *Nco*I, *Not*I, *Pst*I, *Spe*I, *Sph*I, *Pvu*II, *Nar*I. The expected fragments and sizes could be found, verifying the clone as a correct pEpo/neo. Every single nucleotide of the whole cassette (SV40early_promoter - **neo gene** - SV40LTpolyA/IVS - SV40early_promoter - **Epo gene** - SV40LTpolyA/IVS) inserted in the pBR322 vector-part is also confirmed by sequencing.

**Example 2 - Construction of plasmid Epo/dhfr**

**1. Construction of p2-dhfr-CDS**

**1.1 Preparation of the dhfr gene**

The dhfr gene used for the vector construction is taken from a mouse cDNA, present in plasmid pLTRdhfr26 (ATCC 37295). The nucleotide sequence of the mouse dhfr cDNA (MUSDHFR) is available as GenBank Accession No. L26316.

The dhfr is amplified from pLTRdhfr26 using primers designed to produce a fragment containing the coding region from the start ATG at position 56 to the stop codon TAA at position 619. As for the amplification of the neomycin resistance gene described above, *Hind*III and *Spe*I sites are introduced in the upstream and downstream amplification primers, respectively. An *Eco*RI site is also introduced into the reverse primer beside the *Spe*I site. The sequence of the oligonucleotides is as follows:

> *Oligo 2004-13:*          length: 39mer
> 5'- ggg gaa gct t**a**t ggt tcg acc att gaa ctg cat cgt cgc - 3'          SEQ. ID. No. 33
> *5' Hind*III*:* aagctt - A (= pos. 56 in MUSDHFR) TGgttcgaccattg          SEQ. ID. No. 34

> *Oligo 2004-14:*          length: 42mer
> 5'- ggg gaa ttc act agt tag tct ttc ttc tcg tag act tca aac - 3'          SEQ. ID. No. 35
> 5' *Eco*RI / *Spe*I:
> gaattc actag - **t** (= pos. 619 in MUSDHFR) tagtctttcttctcgtagacttcaaact          SEQ. ID. No. 36

The amplification product of primers 2004-13 + 2004-14 is prepared by PCR using *Pwo* polymerase (Roche Diagnostics), as described above. The resulting DNA fragment of 588 bp is purified using DNA isolation columns, digested with *Hind*III and EcoRI and gel-purified.

**1.2 Preparation of p1-dhfr-CDS**

The amplified *Eco*RI-*Hind*III dhfr gene fragment is ligated to the *Eco*RI-*Hind*III vector fragment from pSV2-neo using Ligation Express (Clontech), and transformed into an *E.coli* host. Transformants are-selected-by-growth on LB medium supplemented with 50 mg/l ampicillin. Plasmid DNA from transformants is isolated and purified from colonies on LB medium supplemented with 50 mg/l ampicillin.

Plasmid DNA is isolated from clones and checked by restriction analysis using *Eco*RI plus *Sca*I (3 fragments of size 2225 bp, 514 bp and 473 bp).

Plasmid DNAs showing the expected fragments are further checked by sequencing relevant parts of the constructs. A plasmid DNA containing a verified SV40early promoter and dihydrofolate reductase gene is designated as p1-dhfr-CDS. The analysis of the sequences revealed one deviation within the dhfr gene from the sequence published in MUSDHFR, specifically a change from T to C at position 451 of the MUSDHFR sequence. Subsequent sequencing showed that this change is also present in the source plasmid. However the resulting change does not cause a change in the amino acid sequence encoded by nucleotide sequence since CTT and CTC both encode leucine.

**1.3 Preparation of p2-dhfr-CDS**

p1-dhfr-CDS plasmid DNA is digested using *Eco*RI + *Spe*I. The resulting linearized fragment is purified and ligated with the amplified fragment containing SV40LTpolyA/IVS (described above). Following transformation and selection, resulting plasmids are analyzed by restriction analysis using *Acc*I (3 fragments of 2994, 855 and 216 bp). A few are selected and additionally analyzed using *Hinc*II (2 fragments of 3466 bp and 599 bp, respectively), *Afl*III (2 fragments of 2872 bp and 1193 bp, respectively) and *Bgl*I (2 fragments of 2371 bp and 1694 bp, respectively).

A plasmid DNA showing all the expected fragments in the correct sizes is further checked by sequencing. A verified plasmid is designated as p2-dhfr-CDS.

### 2. Construction of pEpo/dhfr

**2.1 Preparation of pEpo/dhfr 21**

p5 plasmid DNA is digested with *Bam*HI and *Eco*RI and the resulting 1792 bp fragment representing the cassette of SV40promoter-Epogene-SV40terminator is gel-purified.

Plasmid p2-dhfr-CDS is also digested with *Bam*HI and *Eco*RI and the linearized vector is gel purified and eluted using Supelco spin columns. Additionally the DNA is dephosphorylated using alkaline phosphatase and purified with Amicon Micropure enzyme removers.

Both fragments, the 4053 bp p2-dhfr-CDS vector and the 1792 bp cassette from p5, are ligated (Ligation Express, Clontech) and transformed into *E.coli* SURE. Transformants colonies grown on LB medium supplemented with 70 mg/l ampicillin are hybridized using Epo gene (PCR-product) as a probe. Plasmid DNA is isolated from various positive clones and analyzed by digestion using restriction endonuclease *Nco*I.

A clone showing the expected fragments (*Nco*I: 4085 bp and 1760 bp) is selected and designated as pEpo/dhfr-21. For additional purification the DNA is retransformed into *E.coli* SURE (see above) and plasmid DNA prepared using a "Midi-prep" procedure (Qiagen) from a culture inoculated by a single colony (pEpo/dhfr-21-1).

Restriction analysis is performed using the following enzymes: *Bam*HI, *Hind*III, *Eco*RI, *Nco*I, *Not*I, *Pst*I, *Spe*I, *Sph*I, *Pvu*II, *Nar*I. All the expected fragments and sizes could be found, verifying the clone as a correct pEpo/dhfr-21.

**2.2 The final construction of pEpo/dhfr**

In the same way as for pEpo/neo the upstream region of the Epo gene in Epo/dhfr-21 is changed at position -3 referred to the start ATG. An additional nucleotide A is introduced to result in the purine base G at position -3 from start ATG. The Epo gene is reamplified as described in Example 1, section 4.2.

pEpo/dhfr-21 plasmid DNA is digested with *Ksp*I and *Not*I, to remove the Epo gene. The 5259 bp fragment is then gel-purified.

Both prepared DNAs are ligated to each other (Ligation Express, Clontech). Plasmid DNA from transformants is isolated and purified from colonies on LB medium supplemented with 70 mg/l ampicillin. DNAs are analyzed by restriction using NcoI in a first screening.

A positive clone is selected to isolate DNA using a "Midi prep" procedure (Qiagen). An extended restriction analysis is performed using *Bam*HI, *Hind*III, *Eco*RI, *Nco*I, *Not*I, *Pst*I, *Spe*I, *Sph*I, *Pvu*II, *Nar*I. The expected fragments and sizes could be found, verifying the clone as a correct pEpo/dhfr.

Every single nucleotide of the whole cassette (SV40early promoter - **dhfr gene** - SV40LTpolyA/IVS - SV40early promoter - **Epo gene** - SV40LTpolyA/IVS) inserted in the pBR322 vector-part is also confirmed by sequencing.

**Example 3 - Recombinant CHO-cells generated from pEpo/neo and pEpo/dhfr**

$1\text{-}5 \times 10^4$ cells per $cm^2$ are seeded in $25 cm^2$ T-flask bottles or 96-well plates the day before the lipofectin transfection is performed. The two plasmids are mixed at the ratio of 50:1 = Epo/neo:Epo/dhfr and allowed to adsorb to the lipofectin reagent (GIBCO/BRL) according to the manufacturer's protocol.

In brief, we used 0.25 $\mu$g DNA/$cm^2$ and 1.5 $\mu$l lipofectin-reagent/$cm^2$ and adjusted this DNA/lipid cocktail to 200 $\mu$l/$cm^2$ cell layer. Then the cells are overlaid with the transfection cocktail for four hours in serum-free DMEM, before the DNA-containing medium is replaced with cultivation medium. After cultivation for 24 hours in the serum-containing medium we switched to selection medium. Transfected cell-pools are first cultivated in selection medium to confluence and then in amplification medium ($4.8 \times 10^{-8}$ M MTX) before screening the cell culture supernatants by ELISA for Epo production. Highest producers are determined, the MTX concentration increased two-fold and best producers used for further cultivation. 7 recombinant cell pools are selected and comparison made of the growing properties, the Epo productivity, the protein pattern (by western blot analysis), the Epo functionality and the chromosomal stability.

Transfection in T25-flasks is carried out with 2.5 $\mu$g pEpo/neo, 0.05 $\mu$g pEpo/dhfr and 15 $\mu$l lipofectin (09/T25/1 and 09/T25/2) per T25-flask and with 2 $\mu$g pEpo/neo, 0.4 $\mu$g pEpo/dhfr and 15 $\mu$l lipofectin (09/T25/3 and 09/T25/4) per T25-flask.

Additionally five plates are each transfected with 10 $\mu$g pEpo/neo, 0.2 $\mu$g pEpo/dhfr and 60 $\mu$l lipofectin per plate (09/96/1 - 09/96/5), five plates with 8 $\mu$g pEpo/neo, 1.6 $\mu$g pEpo/dhfr and 60 $\mu$l lipofectin per plate (09/96/6 - 09/96/10). Plates 11 and 12 are transfected with 6.25 $\mu$g pEpo/neo, 0.08 $\mu$g pEpo/dhfr and 37.5 $\mu$l lipofectin each.

In brief, 0.25 $\mu$g DNA/$cm^2$ and 1.5 $\mu$l lipofectin-reagent/$cm^2$ are used and this DNA/lipid cocktail adjusted to 200 $\mu$l/$cm^2$ cell layer.

The series of transfections is mainly done in microtitre plates since previous experiments show that the number of clones in one cultivation unit is maximum three to five. This means easier isolation of a monoclonal transfectant than isolation from hundreds of clones in the T-flasks. Table 1 describes the number of clones per 96-well plate and the ELISA titers with and without amplification pressure. Transfected cell pools are first cultivated in selection medium to confluence and then in amplification medium ($4.8 \times 10^{-8}$ M MTX) before screening the cell culture supernatants by ELISA for Epo production. Approximately 1000 growing wells are screened, and 50 such cultures tested for specific Epo-productivity with increased MTX concentration. Highest producers are determined, the MTX concentration increased two-fold and best producers used for further cultivation.

The selection and first amplification steps are done in the 96-well plate and after screening all clones, growing in $4.8 \times 10^{-8}$ M MTX, 7 clones are selected, designated 09/96/1 F5, 09/96/3D5, 09/96/3H5, 09/96/5D4, 09/96/5H1, 09/96/6C5 and 09/96/7E6, and their growing properties, Epo productivity, protein pattern in western blots, Epo functionality tests and chromosomal stability compared.

The cell doubling time seems to be the same for all clones and they can be split 1:2 to 1:5 twice a week. Enhancing the MTX concentration from $9.6 \times 10^{-8}$ M to $1.9 \times 10^{-7}$ M also improves the productivity, while further doubling the MTX concentration does not influence the ELISA value. So subcloning is performed at $3.8 \times 10^{-7}$ M MTX. Immunofluorescence is analyzed at $1.9 \times 10^{-7}$ M MTX where the single cultures do not differ significantly.

[0178] - Cell morphology is compared by light microscopy and Coulter Counter nucleus DNA analysis. Clones 09/96/7E9, 09/96/6C5, 09/96/5H1, 09/96/5D4 and 09/96/3D5 feature the same nucleus size distribution as the host cell line CHO-DHFR$^-$. In contrast, cell lines 09/96/1 F5 and 09/96/3H5 have larger nuclei. It is known from previous experiments that this results from an extended number of chromosomes. It is therefore decided to use clone 09/96/3D5 for further stabilization.

The functionality test for Epo on TF-1 cells gives the same slope for all seven culture supernatants compared with the recombinant pharmaceutical product.

The recombinant protein is tested by SDS PAGE and western blotting at each MTX concentration and only minor changes are found in any of the recombinant culture supernatants. The clones produce Epo.

## Summary and Discussion

The selection of a recombinant, Epo expressing CHO-cell line from the construction of eukaryotic expression vectors up to the transfection of mammalian cells and isolation of polyclonal Epo expressing cell pools is described. The analytical basis is set mainly with ELISA, immunofluorescence, western blotting and *in vitro* functionality tests. All these methods are established in concentration ranges that are capable of screening low producing cell pool culture supernatants with only ng/ml amounts as well as more stabilized recombinant cells.

A recombinant CHO-pool is generated, in which the gene copy number is amplified stepwise with up to $3.8 \times 10^{-7}$ M MTX. These cells can be split 1:3 to 1:4 twice a week and each time elevated levels of Epo are detected in ELISA.

## Example 4 - Further selection of a recombinant cell-line

Recombinant cell-pool 09/96/3D5 is used for further stabilization. MTX concentration is increased stepwise to 0.38 $\mu$M MTX. At this amplification level recombinant 3D5 cells are subcloned with 10 and 20 cells per well. Screening of culture supernatants of wells with single clones is performed by ELISA. Table 2 shows the subcloning conditions and efficiencies of recombinant cell-pool 3D5 in the presence of 0.38 $\mu$M MTX. 300 supernatants of-single-clones are tested. Clones-that-have elevated Epo titers four days after passaging are selected with 0.77 $\mu$M MTX in 24 well plates.

Seven of these clones are conserved in liquid nitrogen and selected for further amplification of gene copy number by increasing MTX concentration to 1.54 $\mu$M.

Table 3 compiles the plating conditions and efficiencies of the second round of stabilization. Here the clones 09/96/3D5/1H9 and 09/96/3D5/18E5, are subcloned a final time with 1.54 $\mu$M MTX. The cell counts per well are reduced to 4 cells. 260 single clones are screened of which more then twenty clones of each subcultivation are transferred to T-flasks and screened for specific productivity. The final production clones are settled by criteria such as specific expression rate, growth conditions and nucleus size distribution. Clones showing tetraploidy are discarded because of the experience that such cells tend to show complicated growth patterns in bioreactors. After screening the following six subclones (four 1 H9 and two 18E5 subclones) are chosen, which are frozen in liquid nitrogen.

| | | |
|---|---|---|
| 09/96/3D5/1H9/4C2 | 09/96/3D5/1H9/6C2 | 09/96/3D5/1H9/6D4 |
| 09/96/3D5/1H9/15B4 | 09/96/3D5/18E5/7A6 | 09/96/3D5/18E5/15C3 |

## Example 5 - Adaptation to serum-free cultivation medium

The final six recombinant cell-lines from Example 4 are chosen for adaptation to serum-free cultivation conditions after the last subcloning step.

Cells are seeded in the 7.- 12. passage after subcloning with approximately $5 \times 10^4$ cells/cm$^2$ into T25-flasks and are cultivated 3-4 days to confluence. At this time point the medium is replaced completely with serum-free adaptation medium and afterwards 80% of the medium is renewed daily. All suspended cells are returned to the culture. After the adaptation time, when nearly all cells grew in suspension, the clones are passaged twice a week and cultivated as suspension-culture.

Clones are cultivated for 11 -13 passages in serum-free adaptation medium before cryopreservation. Six ampoules with $5 \times 10^6$ cells each are frozen of every cell-line in liquid nitrogen with serum-free freezing medium. After thawing, the clones are cultivated in serum-free production medium. Analytical characterization to select for the production clone is done with supernatants in the second or third passage after thawing.

Analytical tests included:

Specific growth rate [$\mu$] - ($\mu$ = ln ($X_2/X_1$) /days)
Specific productivity [$q_P$] - ($Q_P$ = product-generation x $10^6$ / (cell counts x days))
Western blot
Isoelectric focussing
DNA content and stability
Clone stability

All six cell-lines could be grown in serum-free growth media and are split twice a week. Cryopreservation is also performed without serum and after thawing the cultivation medium is switched to serum free production medium. This formulation is enriched in glucose and amino acids.

After 5 passages different protein and cellular parameters are determined and one production clone (09/96/3D5/1 H9/6C2; abbreviated 6C2) and one back up clone (09/96/3D5/1H9/4C2; abbreviated 4C2) selected.

**Example 6 - Comparison of the recombinant cell-lines**

Growth properties of the six cell-lines from Examples 4 and 5 are calculated over several weeks by determining the cell counts in culture as well as splitting ratios during passaging. The Epo productivity is tested by ELISA. From that data the specific productivity and specific growth rate as described above are calculated.

Table 4 summarizes the data received under standard cultivation conditions with a splitting ratio of 1:3 after three days cultivation.

Cell counts (measured by Coulter Counter) after splitting and after additional three days are shown.

SDS-PAGE under reducing conditions

The supernatants of the six cell-lines are separated by SDS-PAGE and compared for differences in the molecular weight. The six supernatants indicate identical SDS patterns with a smear, commonly seen in such highly glycosylated proteins (data not shown).

The comparable commercial available product migrates as a more distinct band probably arising from separating distinct bands during down stream processing.

IEF-Western blot

The IEF-western blot analysis should reflect potential microheterogeneities of the glycoproteins. According to the amount of protein that is loaded on the gel to fourteen bands become visible. There is one characteristic double-band seen on the western blot approximately in the middle of the gel; the next band down under this double-band is defined as band number one and 9 to 10 bands are visible in this acidic part of the gel. The comparable commercial product gave four major bands that correspond to band number six to nine in the heterogeneous product.

DNA content of recombinant cells

The DNA content is proportional to the numbers of chromosomes of cell-lines. The stability of a recombinant cell-line is in part influenced by the chromosomal count and the identity of DNA content is verified by comparison to the host cell-line (CHO dhfr$^-$).

**Summary and Discussion**

The isolation of recombinant, Epo expressing CHO cell-lines is described herein. After two rounds of subcloning six cell-lines are compared for different properties as the basis for the designation of one final production clone. The analytical basis is mainly ELISA, western blotting and IEF tests as well as DNA measurement by FACS analysis.

The western blot pattern of the recombinant culture supernatants shows several additional lower molecular weight bands compared to the commercial purified protein. One explanation is that these additional bands represent isoforms which are removed during the down stream processing leading to the commercial product compared. Another possibility that artificial bands are being detected due to incomplete uptake of SDS. Isoelectric focussing gives identical isoform-distribution for all cell culture supernatants, irrespective of their Qp.

The best producing clone and easiest-handling is clone 6C2 which is chosen as production clone. As back-up clone 4C2 is chosen. Both clones can be propagated in roller bottles.

**Example 7 - Cultivation of CHO cells in T-Flasks**

Recombinant human Erythropoietin is produced in a Chinese hamster ovary cell line (CHO) under serum free conditions in T-Flasks. The culture is seeded with a $2.67 \times 10^5$ cells/mL. After a three day incubation period a final cell density of $9.35 \times 10^5$ cells/mL is reached (=> $\mu$ = 0.42 days$^{-1}$).

Examples 1 to 6 describe the preparation of a number of CHO clones which express Epo. Of the six clones obtained in Examples 4 and 5, clone CHO 6C2 is chosen due to its superior high cell specific productivity and its high specific growth rate.

**Example 8 - Cultivation of CHO cells in a bioreactor**

The CHO cell line 6C2 is cultivated in Fed-Batch (T43C6C2) mode in a 150 L bioreactor. Using a cell culture medium consisting of amino acid-supplemented 50:50 DMEM/Hams F12 and containing 0.25% of a plant peptide, 0.1% lutrol, 1.54 $\mu$M methotrexate (MTX), 4. g/L glucose, 2.5 g/L NaHCO$_3$, ethanolamine, ferric citrate, ascorbic acid and sodium selenite. The medium did not contain any expensive functional proteins (recombinant or from natural sources). Components derived from an animal origin are present. The cells are seeded at around $5\times10^5$ cells/mL in 56 L medium. The pO$_2$ is set to 50% air saturation, temperature to 37°C and the pH to 7.0 and kept constant during the course of the fermentation.

The glucose concentration is kept above 1g/L. After 4 days the reactor is filled to 150 L with fresh medium. After day 9 the batch is extended by adding 1875 mL of a nutrient concentrate containing amino acids, a carbohydrate and a plant derived peptone. After 10 days another 1875 mL of the nutrient concentrate are added. Two days later (day 12) the supernatant containing erythropoietin is harvested.

**Example 9 - Production of erythropoietin in a bioreactor without methotrexate**

The CHO cell line 6C2 is cultivated in Fed-Batch (Kamp 4 B5-1 and 2) mode in a 5-L bioreactor. The medium is as in Example 9. The first bioreactor (Kamp 4 B5-1) is set up with 1.54 $\mu$m MTX in the medium, and the second (Kamp 4 B5-2) without MTX.

The glucose concentration is kept above 1 g/L. The cells are seed at around $5\times10^5$ cells/mL in 1250-mL medium. The pO$_2$ is set to 50% air saturation, temperature to 37°C and the pH to 7.0 and kept constant during the course of the fermentation. After 2 days the reactor is filled to 5 L with fresh medium. After day 6, 7, 8, 9 and 10 the batch is extended by adding 50 to 122-mL of a nutrient concentrate containing amino acids, a carbohydrate and a plant derived peptone. On day 11 the supernatant containing erythropoietin is harvested.

The cultivation without methotrexate is found to be superior due to the better glycosylation pattern.

**Example 10 - Production of Erythropoietin in a bioreactor with enriched medium**

The CHO cell line 6C2 is cultivated in Fed-Batch (Kamp 11 B5-1 and 2) mode in a 5 L bioreactor. Bioreactor 1 is operated as in Example 10 (Kamp 4 B5-2). In bioreactor 2 a cell culture medium is used consisting of an enriched amino acid supplemented 50:50 DMEM/Hams F12 and containing a 0.325% of a plant peptide, 0.1% lutrol, 6.4 g/L glucose, 2.5 g/L NaHCO$_3$, ethanolamine, ferric-citrate, ascorbic acid and sodium selenite and 0.6 g/L phosphate. The cells are seed at around $5\times10^5$ cells/mL in 1250 mL medium. The pO$_2$ is set to 50% air saturation, temperature to 37°C. The pH is set to 7.1 at the beginning. During the course of the fermentation it is reduced step wise to 6.9.

Over the course of the cultivation the glucose concentration in bioreactor 2 is kept between 3 to 4 g/L. After 2.5 days the reactor is filled to 5 L with fresh medium. After day 6, 7, 8, 9 and 10 the batch is extended by adding an enriched nutrient concentrate containing amino acids, a carbohydrate and a plant derived peptone. On day 11 the supernatant containing Epo is harvested.

The cultivation with a nutrient enriched medium (amino acid, glucose, plant peptone and phosphate) as well as the pH-shift from 7.1 to 6.9 is found to more than double the final Epo concentration at a comparable glycosylation profile.

### Example 11 - Production of erythropoietin in a bioreactor lacking components derived from animals

The CHO cell line 6C2 is cultivated in Fed-Batch (Kamp 17 B5-1 and 3) mode in a 5-L bioreactor. All parameters are set as in Example 10 (Kamp 4 B5-2) if not otherwise noted. In bioreactor 2, a cell culture medium is used which does not contain any components derived from animals. For example the amino acid tyrosine or cysteine, which are typically derived from an animal (like salmon or human hair) have been replaced by synthetic amino acids.

After 2.5 days the reactor is filled to 5 L with fresh medium. After day 5, 6, 7, 8 and 9 the batch is extended by adding a nutrient concentrate containing amino acids, a carbohydrate and a plant derived peptone. On day 9 to 10 the supernatant containing erythropoietin is harvested.

A medium not containing any components of animal origin is found to yield a comparable final Epo concentration. However the culture grows slower and needs an additional nutrient concentrate addition.

### Example 12 - Production of Erythropoietin in a bioreactor with enriched medium (vitamins, trace elements)

The CHO cell line 6C2 is cultivated in Fed-Batch (Kamp 12 C) mode in a 10 L bioreactor. The bioreactor is operated as in Example 11 (Kamp 11 B5-2) with the following exceptions:

A cell culture medium is used consisting of an enriched amino acid supplemented 50:50 DMEM/Hams F12 and containing a 0.325% of a plant peptide, 0.1 % lutrol, 6.4 g/L glucose, 2.5 g/L $NaHCO_3$, ethanolamine, ferric-citrate, vitamins, trace elements and sodium selenite and 0.6 g/L phosphate. The content in the concentrate is doubled and enriched with vitamins.

The cells are seeded at around $5 \times 10^5$ cells/mL in 4500 mL medium. The $pO_2$ is set to 50% air saturation, temperature to 37°C. The pH is set to 7.1 at the beginning. During the course of the fermentation it is reduced step wise to 6.9.

Over the course of the cultivation the glucose concentration in the bioreactor is kept between 3 to 4 g/L. After 3 days the reactor is filled to 10 L with fresh medium. After day 6, 7, 8, 9, 10, 11 and 12 the batch is extended by adding the enriched nutrient concentrate. On day 13 the supernatant containing erythropoietin is harvested.

### Example 13 - Isolation of Epo

### Cell Separation

Recombinant human erythropoietin is produced in a Chinese hamster ovary cell line (CHO) under serum free conditions by discontinuous fed batch fermentation. After fermentation (4x ca. 1+2 batch mode expansion stages in 2 different bioreactors) the harvest broth with about 200 - 300 mg rhEpo per L is cooled down to 2-8°C and without any interim storage period clarified first by centrifugation via disc stack separator then subsequently by depth (Seitz Bio10 or Cuno A90M08, throughput ca. 300 L/m$^2$ filter area) and $0.2\mu$ filtration (PP Polygard 0,1$\mu$m, Sartobran P, Sartorius or Duropore 0.22$\mu$, Millipore).To avoid high cell lysis and consequently high product contamination with HCPs (host cell proteins) it is important first to harvest at an optimal time point (ca. 12 days in main culture, oxygen consumption stagnant) and second to use a cell separation equipment specially designed for separation of fragile eukaryotic cells e.g. CSC6 (6000 m$^2$ ECA, 15500 xg, ca. 200 L/h, Westfalia) with hydrohermetic feed inlet or BTPX 250 (11000 m$^2$ ECA, 13000 xg, 300 L/h, Alfa Laval) with gentle disc inlet and porcupine outlet. Comparison of different separation techniques including tangential flow filtration and centrifugation reveals differences in the cell lysis by shear stress (measured by release of the intracellular marker enzyme LDH). The centrifugation gives the most gentle separation (<3U LDH/mg rhEpo) and is to be preferred.

In the alternative, only the centrifugation via disc stack separator (without the depth filtration step) as described above is used for the cell separation step. In another alternative, the depth filtration step (without the centrifugation step) as described above is used.

### Capture by Anion Exchange (AEX) Chromatography

After clarification the crude supernatant is diluted with approx. 3 vol. of water to reach a final conductivity of less than or equal to 5 mS/cm and adjusted to pH 7.5 with Tris base, before applying on the AEX-capture resin.

The used AEX-column has a bed height of about 10 - 20 cm and is packed with a Q Ceramic HyperD F (Biosepra) with good flow characteristics. It is equilibrated with 20 mM Tris pH 7,5 and 50 mM NaCl. The diluted cell supernatant is then loaded (10 -15 mg rhEpo per mL resin) on the column at a flow rate of 4 - 8 cm/min and the column is washed with 10 -15 column volumes (CV) with equilibration buffer. The product is eluted by step elution achieved by changing to a higher conductivity buffer, 20 mM Tris pH 7,5 with 150 mM NaCl. The peak fractions are pooled and give a yield of about 50-60%.

In an alternative, the fraction pool is concentrated by ultrafiltration to reduce the intermediate volume and to standardise the following precipitation conditions. Preferably a 5 to 10 kDa cutoff membrane is used and a target product concentration of about 20 mg/ml is adjusted.

## Ammonium Sulfate Precipitation

The capture pool from the previous step is typically further purified by precipitation of the contaminating host cell proteins with 2.4 M $(NH_4)_2SO_4$. At this AS-concentration almost no product is found in the precipitate leaving a pure HCP free supernatant which has to be diluted before the following RPC purification to < 240 mM $(NH_4)_2SO_4$ in the RPC load.

The precipitation is performed by adding 1.5 volumes of an ammonium sulfate -stock solution (4M $(NH_4)_2SO_4$, 20mM Tris pH 7.5) to one volume of capture pool, incubation for 30 min at 10-15°C and separation of the precipitate by depth (Seitz Bio10 or Cuno A90M08) and $0,2\mu$ filtration (Sartobran P, Sartorius or Duropore $0.22\mu$, Millipore) . In case of high elution volume = dilute rhEpo elution pool (< 5 mg rhEpo/ml) the HCP precipitation can be improved by an optional UF-concentration step (10 kDa cutoff).

The ammonium sulfate precipitation is more effective than hydrophobic interaction chromatography.

To reduce the ammonium sulphate content from the prior precipitation step the supernatant containing the product has to be diluted as mentioned above. An alternative is a ultrafiltration/diafiltration step. Preferably a 5 to 10 kDa cutoff membrane is used and a target ammonium sulphate concentration of less than 240 mM and a product concentration of about 30 mg/ml is adjusted. The used buffer for the diafiltration step is 20 mM Tris/HCl pH 7.0.

## Reversed Phase Chromatography

The next purification step is a reversed phase chromatography which is useful in several respects: a) the different isoforms are well separated according to their sugar backbone (highly glycosylated/sialylated elute before less glycosylated/sialylated forms), b) residual host cell proteins are removed with this high performance chromatography and c) the chromatography is a robust step for virus removal as well as for virus inactivation by the organic solvent. Source 30RPC (Amersham Biosciences) is a polymeric resin which can be a) run under medium pressure (<10 bar) and b) sanitized with high concentrations of NaOH. The preferred bed height is between 10 and 15 cm, the recommended load range 8-12 mg rhEpo per ml packed resin.

Before the RPC-step the product containing supernatant needs to be adjusted to a final concentration of less than 0.24 M ammonium sulfate . This conditioning can be performed a) by a subsequent online-dilution step (1 vol rhEpo-supernatant + 4 vol 20 mM Tris/HCl pH7.0+ 5 vol 50 w% ACN in 20 mM Tris/HCl pH 7.0 during the RPC load or to save the expansive organic solvent preferentially again by diafiltration/ concentration (UF with 10 kDa cutoff) against 20 mM Tris/HCl pH7.0 before the loading step. The column has been equilibrated before and washed after the load with 25w% acetonitrile (ACN) in 20 mM Tris pH 7.0. The product is eluted with a linear gradient from 25% to 50% ACN and collected in small fractions (in particular approx. 0.2 CV, in the alternative, approx. 0.3-0.5 CV) pre-filled with 4 volumes of dilution buffer (50 mM Tris pH 7,0) to immediately reduce the solvent concentration, which may induce aggregation and impairs the following AEX chromatography. The fractions of approximately the first half of the elution peak are pooled to give the RPC-pool, which is further processed. This pool contains the isoforms with the favored higher degree of glycosylation. By this fractionation regime, des-O-glycosylated rhEpo product in which the O-glycan is missing at position Ser126, which is present in the cell culture at levels of up to 20%, is removed.

In an alternative method, to induce virus inactivation by exposure to an organic solvent, the fractions are prefilled with 0.5 volumes of 20 mM Tris pH 7.0 instead of 4 volumes of the same buffer as described above and incubated for 20 to 40 minutes or longer. After this incubation step another 3.5 volumes of 20 mM Tris pH 7.0 referring to the original undiluted fraction volume are added and thus, the virus inactivation is stopped.

The fractions with or without virus inactivation are pooled for further purification. Pooling usually starts at 50 -100% of the maximal OD on the ascending site and end approx. with fractions above 70-80% on the descending site. Earlier eluting fractions can contain host cell proteins, whereas later eluting fractions contain less sialylated, less active isoforms. Additionally a CZE analysis can be performed to support pooling for certain isoforms.

### Anion Exchange Chromatography

The diluted RPC-pool is then loaded on a high performance AEX column, which again helps to select for specific isoforms and remove host cell proteins. This time the isoforms are separated according to the isoelectric point, i.e. according to the number of sialic acids which is proportional to the grade of glycosylation. A high performance resin is used, Q-Sepharose HP (Amersham Biosciences), which shows excellent separation efficiency. The bed height is between 15 and 20 cm. All conditions, such as load, gradient and bed height are defined to keep a rather low product concentration, which otherwise leads to a significant post-peak during elution caused by solvent induced aggregation of the product.

The RPC-pool is loaded with 2-4 mg Epo per mL resin on a Q Sepharose HP equilibrated with 20 mM Tris pH 7,0. After a wash step with equilibration buffer, the product is eluted in a 10 CV linear salt gradient from 0 to 300 mM NaCl in equilibration buffer. The elution peak is collected in 0.1 CV or in 0.25 CV fractions and analytical pools are analyzed by CZE to find the right fraction pool, which contains the desired erythropoietin isoforms. The AEX-pool consists typically of the second half of the elution peak, where the highly glycosylated and sialylated isoforms elute.

By using the capillary zone electrophoresis (CZE) as in-process control, it is possible to produce a precisely defined mixture of erythropoietin isoforms even if the source contains only a few highly sialylated isoforms, due to different fermentation conditions or host systems.

The CZE is a high resolution method capable of separating isoforms of different charge. It gives quantitative results on every single isoform in each fraction. This information enables pooling of specific fractions leading to a consistent isoform profile from batch to batch. Typically the early eluting, less sialylated isoforms are omitted by pooling only the later eluting fractions.

### Size Exclusion Chromatography

In a last chromatographic step the AEX-pool is polished by size exclusion, which removes potential dimers and higher aggregates, and performs a buffer exchange for the final formulation. The Superdex 75 prep grade (Pharmacia) used in this step has a good resolution even at higher load volumes up to 15% of the column volume. The preferred bed height is between 60 and 80 cm.

As it is not possible to run the AEX-chromatography of the previous step in a way to achieve high product concentration in the AEX-pool, the pool has to be concentrated before the gel filtration. This is performed by an ultra-filtration step using a 10 kDa UF-membrane leading in an about 10 fold concentrated UF-retentate with approx. 10 mg erythropoietin per mL.

About 3-7 CV% of UF-retentate are directly loaded onto a Superdex 75 pg column preequilibrated with 20 mM Na-phosphate pH 7.0, 75 mM NaCl. After approx. 1 -1.5 CV the product starts to elute from the column and the elution peak is collected to give the SEC-pool.

### Nano-Filtration

To remove a potential virus load an additional dead-end virus-filtration step is implemented. This filtration is performed with a special membrane, designed to remove particles as small as 15 nm, such as the Planova 15N (Asahi). Alternative dead-end nanofiltration units are PALL Ultipor VF Grade DV20 or Millipore Viresolve NFP cartridges or capsules. Especially for small non-enveloped viruses, e.g. parvovirus, there is almost no other tool of virus removal or inactivation.

The sterile filtered SEC-pool is passed over a dead-end filter with a suitable membrane and the filtrate represents the final bulk drug substance. Alternatively the nanofiltration can be inserted between the UF-concentration and the size exclusion chromatography.

All publications mentioned in the above specification are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are apparent to those skilled in molecular biology or related fields are intended to be within the scope of the following claims.

Table 1: recombinant CHO-cells: transfections with Epo/neo and Epo/dhfr

**T25 transfections**

| transfection | number of clones per T25 | Qp, 9.6x10$^{-8}$ M MTX [µg/10$^6$cells/d] |
|---|---|---|
| 09/T25/1 (50:1) | 136 | 0.16 |
| 09/T25/2 (50:1) | 107 | 2.6 |
| 09/T25/3 (5:1) | 459 | 0.14 |
| 09/T25/4 (5:1) | 648 | 0.9 |

**96-well transfections**

| transfection | number of clones per plate | selected clone |
|---|---|---|
| 09/96/1 (50:1) | 47 | 1F5 |
| 09/96/2 (50:1) | 46 | |
| 09/96/3 (50:1) | 50 | 5D5, 3H5 |
| 09/96/4 (50:1) | 52 | |
| 09/96/5 (50:1) | 49 | 5D4, 5H4 |
| 09/96/6 (5:1) | 416 | 6C5 |
| 09/96/7 (5:1) | 556 | 7E9 |
| 09/96/8 (5:1) | 392 | |
| 09/96/9 (5:1) | 427 | |
| 09/96/10 (5:1) | 352 | |
| 09/96/11 (75:1) | 49 | |
| 09/96/12 (75:1) | 60 | 12A9 |

## Amplification of different clones

| clone | Qp [µg/10cells/d] | | |
|---|---|---|---|
| | 9.6x10$^{-8}$ M MTX | 1.9x10$^{-7}$ M MTX | 3.8x10$^{-7}$ M MTX |
| 09/96/1F5 | 11 | 11.4 | 17.1 |
| 09/96/3D5 | 8.4 | 14.4 | 11.4 |
| 09/96/3H5 | 8.2 | 14.1 | 12.9 |
| 09/96/5D4 | 4.9 | 3.8 | 3.6 |
| 09/96/5H1 | 4.7 | 7.1 | 6.7 |
| 09/96/6C5 | 4.2 | 3.8 | 3.6 |
| 09/96/7E9 | 5.5 | 8.8 | 8.9 |
| 09/96/12A9 | 6 | | |

Table 2: Subcloning conditions and plating efficiencies of cell-pool 3D5 with 0.38 µM MTX

| Number of 96 well plates | Number of seeded cells/well | % growing wells | % single clones |
|---|---|---|---|
| 25 (no. 6-30) | 10 | 13% | 77% |
| 5(no. 1-5) | 20 | 24% | 54% |

Table 3: Subcloning conditions and plating efficiencies of Subclone 3D5/1 H9 and 3D5/18E5 with 1.54 µM MTX

3D5/1 H9

| Number of 96 well plates | Number of seeded cells/well | % growing wells | % single clones |
|---|---|---|---|
| 8 (no. 9-16) | 10 | 6.4% | 85% |
| 8 (no. 1-8) | 30 | 19% | 46% |

3D5/18E5

| Number of 96 well plates | Number of seeded cells/well | % growing wells | % single clones |
|---|---|---|---|
| 8 (no. 9-16) | 4 | 15% | 56% |
| 8 (no. 1-8) | 8 | 29% | 44% |

Table 4: Specific productivity and growth properties of recombinant CHO-clones

| | 4C2 | 6C2 | 6D4 | 15B4 | 7A6 | 15C3 |
|---|---|---|---|---|---|---|
| Inoculated cell number [x 10$^5$ cells/ml] | 2.17 | 2.67 | 2.89 | 0.71 | 2.38 | 2.16 |
| Final cell number [x 10$^5$ cells/ml] | 7.27 | 9.35 | 8.8 | 2.61 | 6.41 | 6.15 |
| Specific growth rate µ [days $^{-1}$] | 0.4 | 0.42 | 0.37 | 0.43 | 0.33 | 0.35 |

**Claims**

1. A method for producing a transformed eukaryotic host cell which expresses a recombinant polypeptide of interest which method comprises introducing into a eukaryotic host cell:

    (a) a first polynucleotide vector which comprises (i) a first nucleotide sequence which encodes a recombinant polypeptide of interest, and (ii) a second nucleotide sequence encoding a selectable marker, which second nucleotide sequence is amplified when the host cell is contacted with a selection agent, and
    (b) a second polynucleotide vector having the same nucleotide sequence as the first polynucleotide vector except that the second nucleotide sequence is replaced with a third nucleotide sequence which encodes a different selectable marker;

    the first polynucleotide vector and second polynucleotide vector being integrated into the genome of the host cell.

2. The method of claim 1 wherein the second nucleotide sequence encodes a dihydrofolate reductase polypeptide and the selection agent is methotrexate.

3. The method of claim 1 wherein the recombinant polypeptide of interest is human erythropoietin.

4. The method of claim 1 wherein the third nucleotide sequence encodes resistance to an antibiotic.

5. The method of claim 4 wherein the antibiotic is G-418, neomycin or a different antibiotic of the neomycin group.

6. The method of claim 1 wherein the host cell is a Chinese hamster ovary (CHO) cell.

7. A transformed eukaryotic host cell comprising, integrated into one or more chromosomes:

    (a) a first polynucleotide vector which comprises (i) a first nucleotide sequence which encodes a recombinant polypeptide of interest; and (ii) a second nucleotide sequence encoding a selectable marker, which second nucleotide sequence is amplified when the host cell is contacted with a selection agent and
    (b) a second polynucleotide vector having the same nucleotide sequence as the first polynucleotide vector except that the second nucleotide sequence is replaced with a third nucleotide sequence which encodes a different selectable marker.

8. The host cell of claim 7 wherein the second nucleotide sequence encodes a dihydrofolate reductase polypeptide.

9. The host cell of claim 7 wherein the recombinant polypeptide of interest is erythropoietin.

10. The host cell of claim 7 which is a CHO cell.

11. A method for producing a recombinant polypeptide of interest which method comprises culturing a transformed host cell according to any of claims 7 to 10 under conditions that permit expression of the first nucleotide sequence.

12. The method of claim 11 wherein the host cell is cultured in the presence of a selection agent that causes amplification of the second nucleotide sequence.

13. The method of claim 12 wherein the second nucleotide sequence encodes a dihydrofolate reductase polypeptide and the selection agent is methotrexate.

14. The method of claim 11 wherein the recombinant polypeptide of interest is erythropoietin.

15. The method of claim 11 wherein the expressed recombinant polypeptide of interest is secreted into the culture medium.

16. The method of claim 15, which further comprises recovering the expressed recombinant polypeptide of interest from the culture medium.

17. The method of claim 16 wherein the recombinant polypeptide of interest is erythropoietin.

**18.** A transformed mammalian host cell comprising stably integrated into one or more chromosomes, (a) a polynucleotide sequence which encodes a dihydrofolate reductase polypeptide and erythropoietin, and (b) a polynucleotide sequence, which is distinct from the polynucleotide sequence of (a) and which encodes erythropoietin and a polypeptide which confers resistance to an antibiotic.

**19.** A method for producing recombinant human erythropoietin which method comprises culturing a host cell according to claim 18 under conditions that permit expression of the polynucleotide sequence encoding human erythropoietin.

**20.** The method of claim 19 wherein the host cell is cultured in the presence of methotrexate.

**21.** The method of claim 19 wherein the expressed recombinant polypeptide of interest is secreted into the culture medium.

**22.** The method of claim 21, which further comprises recovering the expressed recombinant polypeptide of interest from the culture medium.

**23.** A composition comprising:

(a) a first polynucleotide vector which comprises (i) a first nucleotide sequence which encodes a recombinant polypeptide of interest, and (ii) a second nucleotide sequence which when integrated into the host cell genome is amplified when the host cell is contacted with a selection agent that causes amplification of the nucleotide sequence; and
(b) a second polynucleotide vector having the same nucleotide sequence as the first polynucleotide vector except that the second nucleotide sequence is replaced with a third nucleotide sequence which encodes a different selectable marker.

**24.** The composition of claim 23 wherein the recombinant polypeptide of interest is human erythropoietin.

**Patentansprüche**

**1.** Verfahren zur Herstellung einer transformierten eukaryotischen Wirtszelle, die ein interessierendes rekombinantes Polypeptid exprimiert, wobei das Verfahren das Einschleusen in eine eukaryotische Wirtszelle umfasst von:

(a) einem ersten Polynukleotidvektor, der umfasst: (i) eine erste Nukleotidsequenz, die ein interessierendes rekombinantes Polypeptid kodiert, und (ii) eine zweite Nukleotidsequenz, die einen selektierbaren Marker kodiert, wobei die zweite Nukleotidsequenz amplifiziert wird, wenn die Wirtszelle mit einem Selektionsmittel in Kontakt gebracht wird, und
(b) einem zweiten Polynukleotidvektor mit der gleichen Nukleotidsequenz wie der erste Polynukleotidvektor, außer dass die zweite Nukleotidsequenz durch eine dritte Nukleotidsequenz ersetzt ist, die einen anderen selektierbaren Marker kodiert;

wobei der erste Polynukleotidvektor und der zweite Polynukleotidvektor in das Genom der Wirtszelle integriert werden.

**2.** Verfahren nach Anspruch 1, wobei die zweite Nukleotidsequenz ein Dihydrofolatreduktase-Polypeptid kodiert und das Selektionsmittel Methotrexat ist.

**3.** Verfahren nach Anspruch 1, wobei das interessierende rekombinante Polypeptid humanes Erythropoietin ist.

**4.** Verfahren nach Anspruch 1, wobei die dritte Nukleotidsequenz Resistenz gegen ein Antibiotikum kodiert.

**5.** Verfahren nach Anspruch 4, wobei das Antibiotikum G-418, Neomycin oder ein anderes Antibiotikum der Neomycingruppe ist.

**6.** Verfahren nach Anspruch 1, wobei die Wirtszelle eine Ovarienzelle des Chinesischen Hamsters (CHO-Zelle) ist.

**7.** Transformierte eukaryotische Wirtszelle, umfassend, integriert in ein oder mehrere Chromosomen:

(a) einen ersten Polynukleotidvektor, der umfasst: (i) eine erste Nukleotidsequenz, die ein interessierendes rekombinantes Polypeptid kodiert; und (ii) eine zweite Nukleotidsequenz, die einen selektierbaren Marker kodiert, wobei die zweite Nukleotidsequenz amplifiziert wird, wenn die Wirtszelle mit einem Selektionsmittel in Kontakt gebracht wird, und

(b) einen zweiten Polynukleotidvektor mit der gleichen Nukleotidsequenz wie der erste Polynukleotidvektor, außer dass die zweite Nukleotidsequenz durch eine dritte Nukleotidsequenz ersetzt ist, die einen anderen selektierbaren Marker kodiert.

8. Wirtszelle nach Anspruch 7, wobei die zweite Nukleotidsequenz ein Dihydrofolatreduktase-Polypeptid kodiert.

9. Wirtszelle nach Anspruch 7, wobei das interessierende rekombinante Polypeptid Erythropoietin ist.

10. Wirtszelle nach Anspruch 7, die eine CHO-Zelle ist.

11. Verfahren zur Herstellung eines interessierenden rekombinanten Polypeptids, wobei das Verfahren das Kultivieren einer transformierten Wirtszelle nach einem der Ansprüche 7 bis 10 unter Bedingungen umfasst, die die Expression der ersten Nukleotidsequenz erlauben.

12. Verfahren nach Anspruch 11, wobei die Wirtszelle in Gegenwart eines Selektionsmittels kultiviert wird, das eine Amplifikation der zweiten Nukleotidsequenz bewirkt.

13. Verfahren nach Anspruch 12, wobei die zweite Nukleotidsequenz ein Dihydrofolatreduktase-Polypeptid kodiert und das Selektionsmittel Methotrexat ist.

14. Verfahren nach Anspruch 11, wobei das interessierende rekombinante Polypeptid Erythropoietin ist.

15. Verfahren nach Anspruch 11, wobei das exprimierte interessierende rekombinante Polypeptid in das Kulturmedium sezerniert wird.

16. Verfahren nach Anspruch 15, welches weiterhin Gewinnen des exprimierten interessierenden rekombinanten Polypeptids aus dem Kulturmedium umfasst.

17. Verfahren nach Anspruch 16, wobei das interessierende rekombinante Polypeptid Erythropoietin ist.

18. Transformierte Säugerwirtszelle, umfassend, stabil integriert in ein oder mehrere Chromosomen, (a) eine Polynukleotidsequenz, die ein Dihydrofolatreduktase-Polypeptid und Erythropoietin kodiert; und (b) eine Polynukleotidsequenz, die sich von der Polynukleotidsequenz von (a) unterscheidet und die Erythropoietin und ein Polypeptid kodiert, das Resistenz gegen ein Antibiotikum vermittelt.

19. Verfahren zur Herstellung von rekombinantem humanen Erythropoietin, wobei das Verfahren das Kultivieren einer Wirtszelle nach Anspruch 18 unter Bedingungen umfasst, die die Expression der humanes Erythropoietin kodierenden Polynukleotidsequenz erlauben.

20. Verfahren nach Anspruch 19, wobei die Wirtszelle in Gegenwart von Methotrexat kultiviert wird.

21. Verfahren nach Anspruch 19, wobei das exprimierte interessierende rekombinante Polypeptid in das Kulturmedium sezerniert wird.

22. Verfahren nach Anspruch 21, welches weiterhin Gewinnen des exprimierten interessierenden rekombinanten Polypeptids aus dem Kulturmedium umfasst.

23. Zusammensetzung umfassend:

(a) einen ersten Polynukleotidvektor, der umfasst: (i) eine erste Nukleotidsequenz, die ein interessierendes rekombinantes Polypeptid kodiert, und (ii) eine zweite Nukleotidsequenz, die, wenn sie in das Wirtszellengenom integriert ist, amplifiziert wird, wenn die Wirtszelle mit einem Selektionsmittel in Kontakt gebracht wird, das eine Amplifikation der Nukleotidsequenz bewirkt; und
(b) einen zweiten Polynukleotidvektor mit der gleichen Nukleotidsequenz wie der erste Polynukleotidvektor, außer dass die zweite Nukleotidsequenz durch eine dritte Nukleotidsequenz ersetzt ist, die einen anderen selektierbaren Marker kodiert.

24. Zusammensetzung nach Anspruch 23, wobei das interessierende rekombinante Polypeptid humanes Erythropoietin ist.

**Revendications**

1. Procédé de production d'une cellule hôte eucaryote transformée qui exprime un polypeptide recombinant d'intérêt ; ledit procédé consiste à introduire dans une cellule hôte eucaryote :

(a) un premier vecteur polynucléotide qui comprend : (i) une première séquence nucléotide qui code pour un polypeptide recombinant d'intérêt, et (ii) une seconde séquence nucléotide qui code pour un marqueur sélectionnable, ladite seconde séquence nucléotide est amplifiée lorsque la cellule hôte est en contact avec un agent de sélection, et
(b) un second vecteur polynucléotide possédant la même séquence nucléotide que le premier vecteur polynucléotide à la différence près que la seconde séquence nucléotide est remplacée par une troisième séquence nucléotide qui code pour un marqueur sélectionnable qui code pour un marqueur sélectionnable différent ;

le premier vecteur polynucléotide et le second vecteur polynucléotide étant intégrés dans le génome de la cellule hôte.

2. Procédé selon la revendication 1, **caractérisé en ce que** la seconde séquence nucléotide code pour un polypeptide de dihydrofolate réductase et l'agent de sélection est le méthotrexate.

3. Procédé selon la revendication 1, **caractérisé en ce que** le polypeptide recombinant d'intérêt est une érythropoïétine humaine.

4. Procédé selon la revendication 1, **caractérisé en ce que** la troisième séquence nucléotide code une résistance à un antibiotique.

5. Procédé selon la revendication 4, **caractérisé en ce que** l'antibiotique est la G-418, la néomycine ou un antibiotique différent appartenant au groupe de la néomycine.

6. Procédé selon la revendication 1, **caractérisé en ce que** la cellule hôte est une cellule ovarienne de hamster chinois (CHO).

7. Cellule hôte eucaryote transformée comprenant, intégrés dans un ou plusieurs chromosomes :

(a) un premier vecteur polynucléotide qui comprend : (i) une première séquence nucléotide qui code pour un polypeptide recombinant d'intérêt, et (ii) une seconde séquence nucléotide qui code pour un marqueur sélectionnable, ladite seconde séquence nucléotide est amplifiée lorsque la cellule hôte est en contact avec un agent de sélection, et
(b) un second vecteur polynucléotide possédant la même séquence nucléotide que le premier vecteur polynucléotide à la différence près que la seconde séquence nucléotide est remplacée par une troisième séquence nucléotide qui code pour un marqueur sélectionnable différent.

8. Cellule hôte selon la revendication 7, **caractérisée en ce que** la seconde séquence nucléotide code pour un polypeptide de dihydrofolate réductase.

9. Cellule hôte selon la revendication 7, **caractérisée en ce que** le polypeptide recombinant d'intérêt est l'érythropoïétine.

10. Cellule hôte selon la revendication 7 qui est une cellule CHO.

11. Procédé de production d'un polypeptide recombinant d'intérêt ; ledit procédé consiste à cultiver une cellule hôte transformée selon l'une quelconque des revendications 7 à 10 sous des conditions qui permettent l'expression de la première séquence nucléotide.

**12.** Procédé selon la revendication 11, **caractérisé en ce que** la cellule hôte est cultivée en présence d'un agent de sélection qui provoque l'amplification de la seconde séquence nucléotide.

**13.** Procédé selon la revendication 12, **caractérisé en ce que** la seconde séquence nucléotide code pour un polypeptide de dihydrofolate réductase et **en ce que** l'agent de sélection est le méthotrexate.

**14.** Procédé selon la revendication 11, **caractérisé en ce que** le polypeptide recombinant d'intérêt est l'érythropoïétine.

**15.** Procédé selon la revendication 11, **caractérisé en ce que** le polypeptide recombinant d'intérêt est sécrété dans le milieu de culture.

**16.** Procédé selon la revendication 15, **caractérisé en ce qu'**il comprend également la récupération du polypeptide recombinant d'intérêt exprimé à partir du milieu de culture.

**17.** Procédé selon la revendication 16, **caractérisé en ce que** le polypeptide recombinant d'intérêt est l'érythropoïétine.

**18.** Cellule hôte de mammifère transformée comprenant, intégrées de façon stable dans un ou plusieurs chromosomes, (a) une séquence polynucléotide qui code pour un polypeptide de dihydrofolate réductase et une érythropoïétine, et (b) une séquence polynucléotide qui est distincte de la séquence polynucléotide de (a) et qui code pour l'érythropoïétine et un polypeptide qui confère une résistance à un antibiotique.

**19.** Procédé de production d'une érythropoïétine recombinante humaine ; ledit procédé consiste à cultiver une cellule hôte selon la revendication 18 dans des conditions qui permettent l'expression de la séquence polynucléotide codant pour l'érythropoïétine humaine.

**20.** Procédé selon la revendication 19, **caractérisé en ce que** la cellule hôte est cultivée en présence de méthotrexate.

**21.** Procédé selon la revendication 19, **caractérisé en ce que** le polypeptide recombinant d'intérêt exprimé est sécrété dans le milieu de culture.

**22.** Procédé selon la revendication 21, consistant également à récupérer le polypeptide recombinant d'intérêt exprimé à partir du milieu de culture.

**23.** Composition comprenant :

(a) un premier vecteur polynucléoticle qui comprend : (i) une première séquence nucléotide qui code pour un polypeptide recombinant d'intérêt, et (ii) une seconde séquence nucléotide qui, lorsqu'elle est intégrée dans le génome de la cellule hôte, est amplifiée lorsque la cellule hôte est mise en contact avec un agent de sélection qui cause l'amplification de la séquence nucléotide ; et
(b) un second vecteur polynucléoticle possédant la même séquence nucléotide que le premier vecteur polynucléotide à la différence près que la seconde séquence nucléotide est remplacée par une troisième séquence nucléotide qui code pour un marqueur sélectionnable différent.

**24.** Composition selon la revendication 23, **caractérisée en ce que** le polypeptide recombinant d'intérêt est l'érythro-poïétine humaine.

**EP 1 453 966 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5856298 A

- WO 8704462 A

**Non-patent literature cited in the description**

- **Takeuchi ; Kobata.** *Glycobiology,* 1991, vol. 1 (4), 337-346
- **Lee et al.** *J. Biotech,* 1999, vol. 69, 85-93
- **Sambrook et al.** Molecular Cloning: A Laboratory Manual. Cold Spring Harbor Laboratory Press, 1989
- **Ausubel et al.** Short Protocols in Molecular Biology. John Wiley & Sons, Inc, 1999
- **Spatola.** Chemistry and Biochemistry of Amino Acids, Peptides and Proteins. Marcel Dekker, 1983, 267
- **Genes VI.** Lewin. Oxford University Press, 1997, 975-978

- **Urlaub ; Chasin.** *PNAS,* 1980, vol. 77, 4216-4220
- **Van der Blick, A.M. et al.** *Cancer Res,* 1988, vol. 48, 5927-5932
- **Broudy et al.** *Archives of Biochemistry and Biophysics,* 1988, vol. 265, 329-336
- **Ghanem et al.** *Preparative Biochemistry,* 1994, vol. 24 (2), 127-142
- **Hammerling et al.** *J Pharm Biomed Anal,* 1996, vol. 14 (11), 1455-69
- **Urlaub et al.** *PNAS,* 1980, vol. 77 (7), 4216-4220